(19) Europäisches Patentamt / European Patent Office / Office européen des brevets

(11) **EP 4 319 618 B1**

(12) **EUROPEAN PATENT SPECIFICATION**

(45) Date of publication and mention
of the grant of the patent:
**28.01.2026 Bulletin 2026/05**

(21) Application number: **21939640.5**

(22) Date of filing: **06.05.2021**

(51) International Patent Classification (IPC):
**A61B 5/02** *(2006.01)*    **A61B 5/145** *(2006.01)*
**A61B 5/369** *(2021.01)*    **A61B 5/00** *(2006.01)*

(52) Cooperative Patent Classification (CPC):
**A61B 5/14553; A61B 5/02108; A61B 5/369;
A61B 5/6803; A61B 5/681**

(86) International application number:
**PCT/CN2021/091906**

(87) International publication number:
**WO 2022/232992 (10.11.2022 Gazette 2022/45)**

(54) **SYSTEM FOR DETERMINING RISK OF STROKE FOR PERSON**

SYSTEM ZUR BESTIMMUNG DES SCHLAGANFALLRISIKOS FÜR EINE PERSON

SYSTÈME PERMETTANT DE DÉTERMINER UN RISQUE D'ACCIDENT VASCULAIRE CÉRÉBRAL POUR UNE PERSONNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**14.02.2024 Bulletin 2024/07**

(73) Proprietor: **Huawei Technologies Co., Ltd.
Shenzhen, Guangdong 518129 (CN)**

(72) Inventors:
• **AYOUBIAN, Leila
Kanata, Ontario 231 (CA)**
• **VISSAC, Virginie
80992 Munich (DE)**
• **LI, Luping
Shenzhen, Guangdong 518129 (CN)**
• **ZHU, Yu
Shenzhen, Guangdong 518129 (CN)**

(74) Representative: **Roth, Sebastian
Mitscherlich PartmbB
Patent- und Rechtsanwälte
Karlstraße 7
80333 München (DE)**

(56) References cited:
WO-A1-2018/213564    WO-A1-2019/046854
CN-A- 110 022 767    US-A1- 2007 273 504
US-A1- 2011 295 621    US-A1- 2017 347 906
US-A1- 2021 106 238    US-A1- 2021 106 238

• TANG SHUJIN ET AL: "Stroke Outcome
Prediction by Blood Pressure Variability, Heart
Rate Variability, and Baroreflex Sensitivity",
STROKE, vol. 51, no. 4, 1 April 2020 (2020-04-01),
US, pages 1317 - 1320, XP093150688, ISSN:
0039-2499, DOI: 10.1161/
STROKEAHA.119.027981

## Description

### TECHNICAL FIELD

The present disclosure relates to a system for determining a risk of stroke for a person and a method for determining a risk of stroke for a person.

### BACKGROUND

The present disclosure is in the field of stroke risk determination. A stroke occurs due to sudden disturbance of blood supply to the brain (Ischemic), or blood vessel ruptures (Hemorrhagic). Ischemic stroke accounts for 87% of all strokes. Approximately 15 million people are affected by stroke annually, of whom 1/3 die and another third are permanently disabled. Chances of surviving stroke are much higher if patients gets early medical assistance within few hours to save brain tissue and improving recovery.

A system for detecting stroke is known from US 2021/106238.

### SUMMARY

In particular, embodiments of the invention base on the following considerations made by the inventors: Ischemic stroke relates to blockage of small blood vessels in the brain that leads to inadequate oxygenation to the brain tissue. This results in loss of cellular membrane potential and subsequent depolarization of neurons and glial cells. The tissue is potentially salvageable if proper blood flow can be restored on time. Time to treatment inversely correlates with better outcomes. Ischemic stroke is a consequence of a variety of pathological conditions, resulting from either systemic cardiovascular diseases or primary cerebrovascular diseases, which, in the great majority, may be considered as hypertensive or hypertension related cases.

During ischemic stroke cerebral blood flow is disrupted by narrowing of vessels caused by atherosclerosis or embolism. Blood flow is such a parameter that elucidates how fast and efficient the oxygen is carried in the blood and supplied to the tissue. Studies show that blood flow is highly sensitive to pathophysiological alteration, thus could be a potential indicator for early detection of many diseases. Near infrared spectroscopy (NIRS) is an appealing technique probing different biological tissues non-invasively and in real time, giving a new set of information known as cerebral autoregulation (CAR), an independent functional outcome predictor of acute ischemic stroke.

The cerebral blood flow reduction is also accompanied by changes in the pattern of brain electrical activities recorded on encephalography (EEG). Additionally interhemispheric spectral asymmetry of EEG rhythms may assist in stroke detection.

A large proportion of strokes are a consequence of chronic hypertension. On the other hand, acute blood pressure (BP) elevation is one of the most common phenomena observed in the first hours and days after stroke onset. Although blood pressure level is one of the most consistent and powerful predictor of stroke it is not the unique determinant. Baroreflex sensitivity (BRS) is an important determinant of cardiac health and proven to be impaired in stroke patient.

There is a need for a non-invasive, ambulatory, portable and low cost system for prediction and detection of stroke, in order for the patient to get quick medical assistance.

In view of the above-mentioned problems and disadvantages, embodiments of the present invention aim to improve determining a risk of stroke of a person. An objective is to provide a system allowing an improved determination of a risk of stroke of a person. In particular, it is an object to provide a system allowing a determination of a risk of stroke of a person, while fulfilling at least one of the above mentioned needs for such a system.

The objective is achieved by the embodiments of the invention as described in the enclosed independent claims. Advantageous implementations of the embodiments of the invention are further defined in the dependent claims.

A first aspect of the present disclosure provides a system for determining a risk of stroke for a person. The system comprises a plurality of sensors, wearable by the person, and a processor. The plurality of sensors is configured to perform non-invasive measurements at the person for obtaining a first parameter dependent on blood pressure of the person, a second parameter dependent on cerebral blood oxygenation of the person, and a cerebral electric activity parameter of the person (when the person wears the plurality of sensors). The processor is configured to obtain, based on results of the measurements performed by the plurality of sensors, the first parameter, the second parameter and the cerebral electric activity parameter. The processor is configured to determine the risk of stroke of the person based on the first parameter, the second parameter and the cerebral electric activity parameter.

Thus the first aspect of the present disclosure proposes to combine cerebral and cardiovascular biomarkers to improve determination of the risk of a stroke of a person, in particular to enhance detection and prediction of a stroke event. This is done by using the first parameter dependent on blood pressure of the person, the second parameter dependent on cerebral blood oxygenation of the person, and the cerebral electric activity parameter of the person for determining the risk of stroke of the person. The system according to the first aspect allows to do this in a non-invasive, ambulatory and portable way. Moreover, by combining the three different parameters the accuracy and reliability of determining the risk of stroke of the person is improved. Namely, combining the different parameters based on results of measurements of the plurality of sensors (sensor fusion) provides complimentary information, enhances robustness and reliability and increases the accuracy of detection and prediction of stroke and, thus, of determining the risk of stroke of the person.

The system is in particular a system for determining the risk of ischemic stroke of a person.

The processor may be part of a mobile device, such as a smart phone, a smart watch or a piece of clothing wearable at the head. A piece of clothing wearable at the head may correspond to a hat, a cap, a headband, glasses (e.g. smart glasses), a facial mask (e.g. sleeping mask, respiratory mask) etc. The mobile device may correspond to any portable device known in the art, such as a portable computer, portable phone (e.g. cellular phone, smart phone), tablet, handheld device etc. The mobile device may comprise at least one sensor of the plurality of sensors. For example, in case of a smart swatch, the smart watch may comprise the processor and/or at least one sensor for measuring blood pressure of the plurality of sensors. The processor and the plurality of sensors may be configured to communicate with each other, wherein the communication may be wireless and/or wire-bound. The processor may be part of a stationary device, such as a stationary computer (e.g. a work station).

The system, in particular the plurality of sensors and the processors, may be configured to continuously monitor the person's condition. Thus, the system may be referred to as continuous monitoring wearable system.

The processor may correspond to hardware and/or software. In case the processor corresponds to mere software, the processor may correspond to a processing software for processing the results of measurements of the plurality of sensors. The processor may correspond to a controller, microcontroller, microprocessor, field-programmable gate array (FPGA), application specific integrated circuit (ASIC) or any combination thereof. Optionally, the processor may be part of at least one of the plurality of sensors. Alternatively, the processor and one or more sensors of the plurality of sensors may form a device, optionally with a common housing.

The processor may be configured to obtain, based on the results of the measurements performed by the plurality of sensors, a time series of at least one, optionally each, parameter of the first parameter, the second parameter and the cerebral electric activity parameter. The processor may be configured to process the parameter by sampling for a time segment (time window) the time series of the parameter and comparing each sample with at least one threshold for the parameter.

The cerebral electric activity parameter of the person may be referred to as "third parameter indicative of cerebral electric activity of the person".

The first parameter may correspond to the baroreflex sensitivity (BRS). The BRS is a function of blood pressure.

The second parameter may correspond to the cerebral oximetry index (Cox). The Cox may be an indicator for cerebral autoregulation (CAR). Therefore, a value of Cox indicating an impaired CAR indicates a risk of stroke. CAR is a measure of cerebral blood flow based on blood oxygenation.

The cerebral electric activity parameter of the person may correspond to the encephalography (EEG) score. The EEG score may be an indicator for electrical brain activity of the person.

In an implementation form of the first aspect, the first parameter corresponds to the baroreflex sensitivity (BRS) of the person. The plurality of sensors may comprise at least one photoplethysmographic (PPG) sensor configured to measure a pulse to pulse interval variability (PPV) and a pulse parameter (PPar) indicative of blood pressure variability. The processor may be configured to compute, based on the PPV and the pulse parameter indicative of the blood pressure variability, the BRS. That is, the BRS may be used as a cardiovascular biomarker by the system for determining the risk of stroke.

The pulse parameter indicative of the blood pressure variability may be a pulse parameter indicative of systolic arterial pressure variability (SAPV). The processor may be configured to compute the BRS by dividing the PPV by the pulse parameter indicative of the blood pressure variability. The at least one PPG sensor may be part of a mobile device wearable by the person, for example a smart watch wearable at the wrist or a piece of clothing wearable at the head.

The pulse parameter indicative of the blood pressure variability may correspond to at least one of pulse up slope (PUS), pulse amplitude (PA), pulse width (PW), and pulse slope transient time (PSTT).

In an implementation form of the first aspect, the second parameter corresponds to the cerebral oximetry index (Cox). The plurality of sensors may comprise at least two near infrared spectroscopy (NIRS) sensors configured to measure cerebral oxygen saturation (COS) and mean arterial blood pressure (MAP). The processor may be configured to compute, based on the COS and the MAP, the cerebral oximetry index.

The processor may be configured to compute the cerebral oximetry index (Cox) by computing the correlation between the COS and the MAP. The at least two NIRS sensors may be configured to be positioned at the head of the person. For example, the at least two NIRS sensors may be part of a piece of clothing wearable at the head of the person, e.g. a hat, cap, headband, glasses (e.g. smart glasses), facial mask (e.g. sleeping mask, respiratory mask) etc. NIRS sensors have a high penetration depths (e.g. higher than PPG sensors) and, thus, are advantageous for measuring COS and MAP.

As mentioned already above, the Cox is an indicator for cerebral autoregulation (CAR). Thus, the CAR (indicated by the Cox) may be used as a cerebral biomarker by the system for determining the risk of stroke.

In an implementation form of the first aspect, the cerebral electric activity parameter corresponds to an encephalography (EEG) score. The plurality of sensors may comprise at least five EEG sensors. The processor may be configured to compute, based on results of measurements performed by the at least five EEG sensors, the EEG score.

The at least five EEG sensors may be configured to measure an electric power of alpha waves, an electric power of beta waves, an electric power of theta waves and/or an electric power of delta waves. In other words, the at least five EEG sensors may be configured to measure an electric power in the alpha frequency band, the beta frequency band, the theta frequency band and the delta frequency band.

The at least five EEG sensors may be configured to be positioned at the head of the person. For example, the at least five EEG sensors may be part of a piece of clothing wearable at the head of the person, e.g. a hat, cap, headband, glasses (e.g. smart glasses), facial mask (e.g. sleeping mask, respiratory mask) etc.

As mentioned already above, the EEG score is an indicator for electrical brain activity. Thus, the electrical brain activity may be used as a cerebral biomarker by the system for determining the risk of stroke.

In an implementation form of the first aspect, the processor is configured to compute, based on the results of the measurements performed by the at least five EEG sensors, at least one of:

- a ratio between a measured power of delta waves and a measured power of alpha waves (DAR),
- a ratio between a sum of the measured power of delta waves and a measured power of theta waves and a sum of the measured power of alpha waves and a measured power of beta waves (DBATR), and
- a pairwise-derived brain symmetry index (PDBSI).

The processor may be configured to compute, based on at least one of the DAR, DBATR and PDBSI, the EEG score.

The processor may be configured to compute the DAR by dividing the measured power of the delta waves by the measured power of the alpha waves. The processor may be configured to compute the DBATR by dividing the sum of the measured power of the delta waves and the measured power of the theta waves by the sum of the measured power of the alpha waves and the measured power of the beta waves. The measured power is an electric power.

At least four EEG sensors of the five EEG sensors form at least two EEG sensor pairs each comprising a respective first EEG sensor for measuring cerebral electric activity in the left cerebral hemisphere of the person and respective second EEG sensor for measuring cerebral electric activity in the right cerebral hemisphere of the person. The respective first EEG sensor and the respective second EEG sensor of each EEG sensor pair are configured to be positioned at corresponding positions of the two cerebral hemispheres of the person. The processor may be configured to compute the PDBSI by computing an absolute value of a difference in measured power at each EEG sensor pair of the at least two EEG sensor pairs between the two cerebral hemispheres.

The processor may be configured to compute the EEG score by combining the DAR, the DBATR and the PDBSI using a classifier. The classifier may be a tree baggers classifier. The processor may be configured to input the DAR, the DBATR and the PDBSI to the tree baggers classifier that generates, based on the DAR, the DBATR and the PDBSI, classification trees. The processor may be configured to combine the generated classification trees to generate a final classification tree for generalizing the DAR, the DBATR and the PDBSI to the EEG score.

Optionally, the EEG score corresponds to one of the DAR, the DBATR and the PDBSI.

In an implementation form of the first aspect, the plurality of sensors may comprise at least one inertial sensor. The processor may be configured to detect, based on results of measurements performed by the at least one inertial sensor, activity of the person. The processor may be configured to discard the obtained first parameter, second parameter and cerebral electric activity parameter, in case of detecting activity of the person.

The at least one inertial sensor may comprise or correspond to at least one of accelerometers and angular rate sensors (e.g. gyroscopes). The at least one inertial sensor may be part of a mobile device, such as a smart watch or a piece of clothing wearable at the head. In particular, it may be part of a device comprising at least one sensor of the plurality of sensors for obtaining the first parameter, the second parameter and the cerebral electric activity parameter of the person.

In an implementation form of the first aspect, the processor is configured to obtain a patient clinical score of the person, and determine the risk of stroke of the person based on the patient clinical score of the person in addition to the first parameter, the second parameter and the cerebral electric activity parameter. The patient clinical score may comprise or correspond to a clinical stroke score from patient history.

The patient clinical score may be for example the CHA2DS2-VASc score. The CHA2DS2-VASc score has the modest discriminatory capacity for ischemic stroke detection. This score is measured by the following parameters: congestive heart failure, hypertension, age ≥75 [doubled], diabetes mellitus, stroke [doubled], vascular disease, age 65 to 74 years, and female sex. The parameters of congestive heart failure, hypertension, diabetes mellitus, vascular disease, age 65 to 74 years, and female sex are weighted by one and the parameters of age ≥75 and stroke are weighted by two (i.e.

doubled) for forming the CHA2DS2-VASc score.

In an implementation form of the first aspect, the processor is configured to compute, based on the first parameter, the second parameter and the cerebral electric activity parameter, a first probability vector to determine the risk of stroke of the person. The first probability vector comprises at least two entries indicating at least two probabilities of at least two classes with regard to risk of stroke. The term "categories" may be used as a synonym for the term "classes". The at least two probabilities indicated by the at least two entries of a probability vector, such as the first probability vector, add up to one respectively one hundred percent (1 respectively 100%).

In an implementation form of the first aspect, for each parameter of the first parameter, the second parameter and the cerebral electric activity parameter, the processor is configured to compute, based on the parameter, a respective second probability vector using a respective classifier, to compute three second probability vectors. Each of the three second probability vectors comprises at least two entries indicating the at least two probabilities of the at least two classes with regard to risk of stroke. The processor may be configured to compute the first probability vector by combining the three second probability vectors using a weighted sum.

In case each probability vector comprises two entries indicating two probabilities of two classes with regard to risk of stroke, the respective classifier may be configured to compute the two probabilities using a probability density function and at least one threshold for the parameter.

The processor may be configured to obtain, based on the results of the measurements performed by the plurality of sensors, a time series of at least one, optionally each, parameter of the first parameter, the second parameter and the cerebral electric activity parameter. The processor may be configured to process the parameter by sampling for a time segment (time window) the time series of the parameter and comparing each sample with at least one threshold for the parameter. The respective classifier is configured to compute, for the time segment, the at least two probabilities indicated by the at least two entries of the respective second probability vector by comparing each sample of the parameter with the at least one threshold for the parameter.

In particular, a respective second probability vector computed, based on a parameter of the first parameter, second parameter and the cerebral brain activity parameter, by a classifier corresponds to a decision computed by the classifier for the parameter. That is, for each parameter of the first parameter, second parameter and the cerebral brain activity parameter, the processor may be configured to input the parameter to the respective classifier, which computes based on the parameter a respective decision in the form of the respective second probability vector. The first probability vector may be referred to as final decision computed, based on the first parameter, second parameter and the cerebral brain activity parameter, by the processor with regard to the risk of stroke of the person.

In an implementation form of the first aspect, the processor is configured to compute the first probability vector by using a trained machine learning model that weights each of the three second probability vectors and sums the three weighted second probability vectors to generate the first probability vector. The machine learning model is trained using training data comprising a plurality of data sets, wherein each data set comprises for a respective time segment one or more samples of the first parameter, the second parameter and the cerebral electric activity parameter associated with a probability vector indicating at least a known risk of stroke for the respective time segment.

The weighting by the trained machine learning model may be optimized using a optimization problem algorithm. In particular, the machine learning model may be trained using the optimization problem algorithm. The optimization problem algorithm may comprise: computing for the one or more samples of the first parameter, the second parameter and the cerebral electric activity parameter of a data set of the training data the three second probability vectors (using for each parameter the respective classifier) and inputting the three second probability vectors to the machine learning model. Further, the algorithm may comprise: receiving the first probability vector generated by the machine learning model based on the three second probability vectors and comparing the generated first probability vector with the probability vector of the data set. Furthermore, the algorithm may comprise: updating the weighting of the machine learning model (performed on the three second probability vectors) based on a difference between the generated first probability vector and the probability vector of the data set. Moreover, the algorithm may comprise: repeating the previous steps of the algorithm with further data sets of the training data until the difference between the respective generated first probability vector and the probability vector of the respective data set is smaller than a threshold for the difference.

The probability vector indicating at least a known risk of stroke for the respective time segment may be referred to as "ground truth" or "ground truth value". For training the machine learning model a majority voting algorithm may be used.

In an implementation form of the first aspect, for each parameter of the first parameter, the second parameter and the cerebral electric activity parameter, the processor is configured to compute, based on the parameter, two or more respective second probability vectors using two or more respective classifiers. The two or more respective classifiers correspond to two or more classifier types. For each classifier type of the two or more classifier types, the processor may be configured to compute a respective third probability vector by combining three respective second probability vectors, computed by the classifier type, using a weighted sum to compute two or more third probability vectors. The processor may be configured to compute the first probability vector by combining the two or more third probability vectors using a weighted sum.

In particular, a respective third probability vector computed for a classifier type, based on the respective three second probability vectors (computed by the classifier type), corresponds to a decision computed for the classifier type. That is, for each classifier type of the at least two classifier types, the processor may be configured to separately input the first parameter, second parameter and the cerebral electric activity parameter to the classifier type. The classifier type computes three respective second probability vectors by computing for each parameter, using the classifier type, the respective second probability vector of the three respective second probability vectors. Moreover, for each classifier type of the at least two classifier types, the processor may be configured to combine the computed three respective second vectors to compute a respective decision of the classifier type in the form of the respective third probability vector.

In an implementation form of the first aspect, the processor is configured to compute the first probability vector by using two trained machine learning models. For each classifier type, a first trained machine learning model of the two models weights each of the three respective second probability vectors and sums the three weighted second probability vectors to generate the respective third probability vector. A second trained machine learning model of the two models weights each of the two or more third probability vectors and sums the two or more weighted third probability vectors to generate the first probability vector. The two machine learning models are trained using training data comprising a plurality of data sets. Each data set comprises for a respective time segment one or more samples of the first parameter, the second parameter and the cerebral electric activity parameter associated with a probability vector indicating at least a known risk of stroke for the respective time segment.

The weighting by each of the two trained machine learning models may be optimized using an optimization problem algorithm. In particular, the two machine learning models may be trained using the optimization problem algorithm. The above description with regard to an optimization problem algorithm is correspondingly valid. Thus, the optimization problem algorithm may comprise: inputting the one or more samples of the first parameter, the second parameter and the cerebral electric activity parameter of a data set of the training data to the at least two classifiers corresponding to at least two classifier types to compute for each classifier type three respective second probability vectors. Further, the optimization problem algorithm may comprise: inputting, for each classifier type, the computed three respective second probability vectors to the first machine learning model to compute the two or more third probability vectors for the two or more classifier types. Furthermore, inputting the two or more third probability vectors, computed based on the data set of the training data, to the second machine learning model. Moreover, the algorithm may comprise: receiving the first probability vector computed by the second machine learning model and comparing the computed first probability vector with the probability vector of the data set. In addition, the algorithm may comprise: updating the weighting of the first machine learning model and the weighting of the second machine learning model based on a difference between the computed first probability vector and the probability vector of the data set. Further, the algorithm may comprise: repeating the previous steps of the algorithm with further data sets of the training data until the difference between the respective computed first probability vector and the probability vector of the respective data set is smaller than a threshold for the difference. For training the two machine learning models a majority voting algorithm may be used.

In an implementation form of the first aspect, each probability vector comprises two entries, wherein a first entry of the two entries indicates the probability of no risk of stroke and a second entry of the two entries indicates the probability of a risk of stroke. Alternatively, each probability vector may comprise three entries. A first entry of the three entries indicates the probability of a probability of stroke smaller than or equal to a first probability threshold. A second entry of the three entries indicates the probability of a probability of stroke greater than the first probability threshold and smaller than or equal to a second probability threshold. A third entry of the three entries indicates the probability of a probability of stroke greater than the second probability threshold.

Alternatively, each probability vector may comprise four entries. A first entry of the four entries indicates the probability of a probability of stroke smaller than or equal to a first probability threshold. A second entry of the four entries indicates the probability of a probability of stroke greater than the first probability threshold and smaller than or equal to a second probability threshold. A third entry of the four entries indicates the probability of a probability of stroke greater than the second probability threshold. A fourth entry of the four entries indicates the probability that the first parameter, the second parameter and/or the cerebral electric activity parameter, used for determining the probability vector, were not suitable for determining the probability vector.

In an implementation form of the first aspect, the system comprises a user interface for

-    informing the person on the determined risk of stroke,
-    allowing the person to correct computation results of the processor, and/or
-    allowing the person to input ground truth information with respect to a risk of stroke. In addition or alternatively, the system may comprise a warning device configured to warn the person in case the determined risk of stroke is greater than a threshold for the risk.

The user interface may comprise or correspond to a display. The display may be a touch display. The display may be a display of a mobile device. The mobile device may correspond to any portable device known in the art, such as a portable computer, portable phone (e.g. cellular phone, smart phone), tablet, handheld device etc. The user interface may be part of a stationary device, such as a stationary computer (e.g. a work station). The user interface may be part of a device comprising the processor of the system. The processor may be configured to communicate with the user interface, wherein the communication may be wireless and/or wire-bound.

The warning device may be part of or correspond to a mobile device. The mobile device may correspond to any portable device known in the art, such as a portable computer, portable phone (e.g. cellular phone, smart phone), tablet, handheld device etc. The warning device may be part of or correspond to a stationary device, such as a stationary computer (e.g. a work station). The warning device may be part of or correspond to a device comprising the processor of the system. The warning device may comprise or correspond to the user interface.

The warning device may be configured to output an audio/audible warning (e.g. an audio signal, such as an alarm or a voice), a visual warning (e.g. a flashing) and/or a tactile warning (e.g. a vibration). Therefore, the warning device may comprise at least one of means for generating an audio stimulus (e.g. at least one loudspeaker), means for generating a visual stimulus (e.g. at least one lighting means, such as at least one LED or another lamp type, and/or at least one display) and means for generating a tactile stimulus (e.g. at least one vibrator). The output by the warning device may be dependent on the severity of the risk of stroke determined by the system. For example, for a medium risk, a LED of the warning device may be activated providing a visual stimulus and for a high risk in addition or alternatively an audio alarm may be output by the warning device.

In order to achieve the system according to the first aspect of the present disclosure, some or all of the implementation forms and optional features of the first aspect, as described above, may be combined with each other.

A second aspect of the present disclosure provides a method for determining a risk of stroke for a person. The method comprises performing non-invasive measurements at the person for obtaining a first parameter dependent on blood pressure of the person, a second parameter dependent on cerebral blood oxygenation of the person and a cerebral electric activity parameter of the person. Further, the method comprises obtaining, based on results of the non-invasive measurements, the first parameter, the second parameter and the cerebral electric activity parameter. Furthermore, the method comprises determining the risk of stroke of the person based on the first parameter, the second parameter and the cerebral electric activity parameter.

The implementation forms and optional features of the system according to the first aspect are correspondingly valid for the method according to the second aspect.

In an implementation form of the second aspect, the first parameter corresponds to the baroreflex sensitivity (BRS) of the person. The method may comprise measuring a pulse to pulse interval variability (PPV) and a pulse parameter indicative of blood pressure variability. Further, the method may comprise computing, based on the PPV and the parameter indicative of the blood pressure variability, the BRS.

The pulse parameter indicative of the blood pressure variability may correspond to at least one of pulse up slope (PUS), pulse amplitude (PA), pulse width (PW), and pulse slope transient time (PSTT).

In an implementation form of the second aspect, the second parameter corresponds to the cerebral oximetry index (Cox). The method may comprise measuring cerebral oxygen saturation (COS) and mean arterial blood pressure (MAP). Further, the method may comprise computing, based on the COS and the MAP, the cerebral oximetry index.

In an implementation form of the second aspect, the cerebral electric activity parameter corresponds to an encephalography (EEG) score. The method may comprise computing, based on results of measurements performed by at least five EEG sensors, the EEG score.

In an implementation form of the second aspect, the method comprises computing, based on the results of the measurements performed by the at least five EEG sensors, at least one of:

- a ratio between a measured power of delta waves and a measured power of alpha waves (DAR),
- a ratio between a sum of the measured power of delta waves and a measured power of theta waves and a sum of the measured power of alpha waves and a measured power of beta waves (DBATR), and
- a pairwise-derived brain symmetry index (PDBSI).

The method may comprise computing, based on at least one of the DAR, DBATR and PDBSI, the EEG score.

In an implementation form of the second aspect, the method may comprise detecting, based on results of measurements performed by at least one inertial sensor, activity of the person. The method may comprise discarding the obtained first parameter, second parameter and cerebral electric activity parameter, in case of detecting activity of the person.

In an implementation form of the second aspect, the method may comprise obtaining a patient clinical score of the person, and determining the risk of stroke of the person based on the patient clinical score of the person in addition to the first parameter, the second parameter and the cerebral electric activity parameter.

In an implementation form of the second aspect, the method may comprise computing, based on the first parameter, the second parameter and the cerebral electric activity parameter, a first probability vector to determine the risk of stroke of the person. The first probability vector comprises at least two entries indicating at least two probabilities of at least two classes with regard to risk of stroke.

In an implementation form of the second aspect, for each parameter of the first parameter, the second parameter and the cerebral electric activity parameter, the method may comprise computing, based on the parameter, a respective second probability vector using a respective classifier, to compute three second probability vectors. Each of the three second probability vectors comprises at least two entries indicating the at least two probabilities of the at least two classes with regard to risk of stroke. The method may comprise computing the first probability vector by combining the three second probability vectors using a weighted sum.

In an implementation form of the second aspect, the method may comprise computing the first probability vector by using a trained machine learning model that weights each of the three second probability vectors and sums the three weighted second probability vectors to generate the first probability vector. The machine learning model is trained using training data comprising a plurality of data sets, wherein each data set comprises for a respective time segment one or more samples of the first parameter, the second parameter and the cerebral electric activity parameter associated with a probability vector indicating at least a known risk of stroke for the respective time segment.

In an implementation form of the second aspect, for each parameter of the first parameter, the second parameter and the cerebral electric activity parameter, the method may comprise computing, based on the parameter, two or more respective second probability vectors using two or more respective classifiers. The two or more respective classifiers correspond to two or more classifier types. For each classifier type of the two or more classifier types, the method may comprise computing a respective third probability vector by combining three respective second probability vectors, computed by the classifier type, using a weighted sum to compute two or more third probability vectors. The method may comprise computing the first probability vector by combining the two or more third probability vectors using a weighted sum.

In an implementation form of the second aspect, the method comprises computing the first probability vector by using two trained machine learning models. For each classifier type, a first trained machine learning model of the two models weights each of the three respective second probability vectors and sums the three weighted second probability vectors to generate the respective third probability vector. A second trained machine learning model of the two models weights each of the two or more third probability vectors and sums the two or more weighted third probability vectors to generate the first probability vector. The two machine learning models are trained using training data comprising a plurality of data sets. Each data set comprises for a respective time segment one or more samples of the first parameter, the second parameter and the cerebral electric activity parameter associated with a probability vector indicating at least a known risk of stroke for the respective time segment.

In an implementation form of the second aspect, each probability vector comprises two entries, wherein a first entry of the two entries indicates the probability of no risk of stroke and a second entry of the two entries indicates the probability of a risk of stroke. Alternatively, each probability vector may comprise three entries. A first entry of the three entries indicates the probability of a probability of stroke smaller than or equal to a first probability threshold. A second entry of the three entries indicates the probability of a probability of stroke greater than the first probability threshold and smaller than or equal to a second probability threshold. A third entry of the three entries indicates the probability of a probability of stroke greater than the second probability threshold.

Alternatively, each probability vector may comprise four entries. A first entry of the four entries indicates the probability of a probability of stroke smaller than or equal to a first probability threshold. A second entry of the four entries indicates the probability of a probability of stroke greater than the first probability threshold and smaller than or equal to a second probability threshold. A third entry of the four entries indicates the probability of a probability of stroke greater than the second probability threshold. A fourth entry of the four entries indicates the probability that the first parameter, the second parameter and/or the cerebral electric activity parameter, used for determining the probability vector, were not suitable for determining the probability vector.

In an implementation form of the second aspect, the method comprises

- informing the person on the determined risk of stroke,
- allowing the person to correct computation results, and/or
- allowing the person to input ground truth information with respect to a risk of stroke. In addition or alternatively, the method may comprise warning the person in case the determined risk of stroke is greater than a threshold for the risk.

The method of the second aspect and its implementation forms and optional features achieve the same advantages as the system of the first aspect and its respective implementation forms and respective optional features.

In order to achieve the method according to the second aspect of the present disclosure, some or all of the implementation forms and optional features of the second aspect, as described above, may be combined with each other.

A third aspect of the present disclosure provides a computer program comprising program code for performing when implemented on a processor, a method according to the second aspect or any of its implementation forms.

A fourth aspect of the present disclosure provides a computer program comprising a program code for performing the method according to the second aspect or any of its implementation forms.

A fifth aspect of the present disclosure provides a computer comprising a memory and a processor, which are configured to store and execute program code to perform the method according to the second aspect or any of its implementation forms.

A sixth aspect of the present disclosure provides a non-transitory storage medium storing executable program code which, when executed by a processor, causes the method according to the second aspect or any of its implementation forms to be performed.

The computer program of the third aspect, the computer program of the fourth aspect, the computer of the fifth aspect and the non-transitory storage memory of the sixth aspect achieve the same advantages as the system of the first aspect and its respective implementation forms and respective optional features.

It has to be noted that all devices, elements, units and means described in the present application could be implemented in software or hardware elements or any kind of combination thereof. All steps which are performed by the various entities described in the present application as well as the functionalities described to be performed by the various entities are intended to mean that the respective entity is adapted to or configured to perform the respective steps and functionalities. Even if, in the following description of specific embodiments, a specific functionality or step to be performed by external entities is not reflected in the description of a specific detailed element of that entity which performs that specific step or functionality, it should be clear for a skilled person that these methods and functionalities can be implemented in respective software or hardware elements, or any kind of combination thereof.

## BRIEF DESCRIPTION OF DRAWINGS

The above described aspects and implementation forms will be explained in the following description of specific embodiments in relation to the enclosed drawings, in which

**Figure 1**    shows an example of a system according to an embodiment of the invention;

**Figure 2**    shows an example of a system according to an embodiment of the invention;

**Figure 3**    shows an example of the arrangement of the plurality of sensors of a system according to an embodiment of the invention, when a person wears the plurality of sensors;

**Figure 4**    shows an example of processing the results of measurements performed by the plurality of sensors of a system according to an embodiment of the invention;

**Figure 5**    shows an example of determining the risk of stroke of a person by computing a first probability vector by a processor of a system according to an embodiment of the invention;

**Figure 6**    shows an example of processing, using a classifier, baroreflex sensitivity (BRS) by a processor of a system according to an embodiment of the invention; and

**Figure 7**    shows an example of determining the risk of stroke of a person by computing a first probability vector by a processor of a system according to an embodiment of the invention.

In the Figures corresponding components are marked by the same reference sign.

## DETAILED DESCRIPTION OF EMBODIMENTS

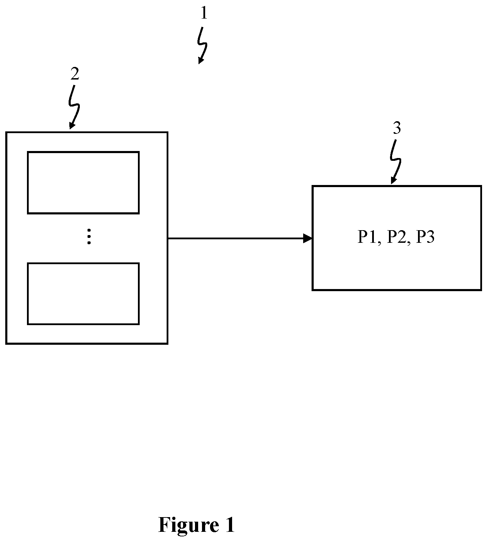

**Figure 1** shows an example of a system according to an embodiment of the invention. The system 1 is a system for determining a risk of stroke for a person. The system 1 comprises a plurality of sensors 2, wearable by the person, and a processor 3. The plurality of sensors 2 is configured to perform non-invasive measurements at the person for obtaining a first parameter P1 dependent on blood pressure of the person, a second parameter P2 dependent on cerebral blood oxygenation of the person, and a cerebral electric activity parameter P3 of the person (when the person wears the plurality of sensors). The processor 3 is configured to obtain, based on results of the measurements performed by the plurality of sensors 2, the first parameter P1, the second parameter P2 and the cerebral electric activity parameter P3. The processor is configured to determine the risk of stroke of the person based on the first parameter P1, the second parameter P2 and the cerebral electric activity parameter P3.

The processor 3 may be referred to as processing circuitry. The processor 3 is configured to perform, conduct or initiate the various operations of the system 1 described herein for determining the risk of stroke of the person. The processor 3 may be configured to perform, conduct or initiate the various operations of the processor of the system according to the first aspect, as described above. The processor 3 may comprise hardware and/or may be controlled by software. The hardware may comprise analog circuitry or digital circuitry, or both analog and digital circuitry. The digital circuitry may comprise components such as application-specific integrated circuits (ASICs), field-programmable arrays (FPGAs), digital signal processors (DSPs), or multi-purpose processors.

The system 1 may further comprise memory circuitry (associated with the processor 3, in particular being part of the processor 3), which stores one or more instruction(s) that can be executed by the processor 3, in particular under control of the software (not shown in Figure 1). For instance, the memory circuitry may comprise a non-transitory storage medium storing executable software code which, when executed by the processor 3, causes the processor 3 to perform, conduct or initiate the operations or methods described herein.

The processor 3 may be part of at least one sensor of the plurality of the sensors 2 (not shown in Figure 1). One or more sensors of the plurality of sensors 2 and the processor 3 may be part of or form a device, optionally with a common housing (not shown in Figure 1). The device may be a mobile device. For example, at least one sensor of the plurality of sensors 2 and the processor 3 may be part of a mobile device, such as a smart watch wearable by the person at the wrist.

For further details of the system 1 of Figure 1 reference is made to the above description of the system according to the first aspect of the present disclosure.

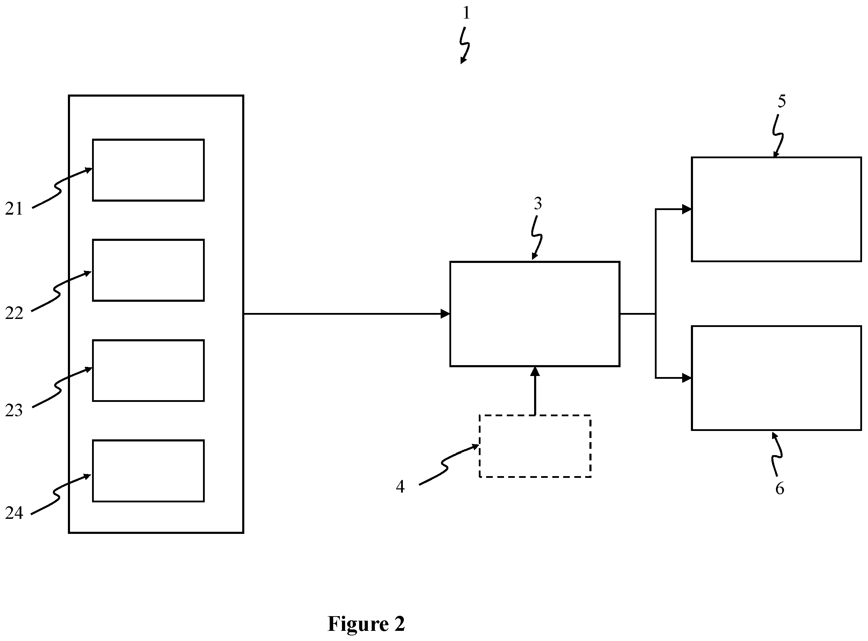

**Figure 2** shows an example of a system according to an embodiment of the invention. The system 1 of Figure 2 corresponds to the system of Figure 1. Therefore, the description of the system 1 of Figure 1 is correspondingly valid for the system 1 of Figure 2. In the following, mainly additional features not shown in Figure 1 are described.

As indicated in Figure 2, the plurality of sensors may comprise or correspond to one or more photoplethysmographic (PPG) sensors 21, two or more near infrared spectroscopy (NIRS) sensors 22, five or more encephalography (EEG) sensors 23 and one or more optional inertial sensors 24. The one or more PPG sensors 21 are provided for obtaining the baroreflex sensitivity (BRS) of the person as the first parameter P1 dependent on blood pressure of the person. The two or more near infrared spectroscopy (NIRS) sensors 22 are provided for obtaining the cerebral oximetry index (Cox) as the second parameter P2 dependent on cerebral blood oxygenation of the person. The five or more EEG sensors 23 are provided for obtaining the EEG score as the cerebral electric activity parameter P3 of the person.

Baroreflex sensitivity (BRS), heart rate variability (HRV) and blood pressure variability are promising biomarkers for stroke risk determination among indices of cardiovascular autoregulation that may be used for risk stratification and diagnosis of stroke. Short and long term HRV monitoring shows insights into prediction of stroke. In acute post-stroke patients a reduction of HRV is often observed. Additionally it is observed that chronic post stroke patients show high systolic blood pressure variability. The baroreflex (also referred to as baroreceptor reflex) is the principal neural mechanism involved in blood pressure regulation. An impairment of the baroreflex may result in a significant dysregulation of blood pressure, leading to an increased blood pressure variability.

BRS is an established tool for assessing autonomic control of the cardiovascular system.

These techniques are noninvasive, low cost and shows the interaction between baroreflex function and cardiovascular parameter. BRS may be defined as the change in the pulse to pulse interval variability (PPV) following a unitary change in blood pressure (BP) and is expressed in ms/mmHg. Baroreflex impairment has been repeatedly shown to be present in acute ischemic within 72 hours. It is an independent predictor for stroke in hypertension patients and modification of BRS may be used as a therapeutic target implication in acute ischemic stroke. BRS values are more impaired in ischemic stroke patients with hypertension than in stroke-free hypertensive patients. Decreased BRS may be found in patients with acute intracerebral hemorrhage and correlated with increased beat-to-beat blood pressure variability. Hypertensive stroke patients with metabolic syndrome have critical BRS values smaller than 3 ms/mmHg (BRS < 3 ms/mmHg), while a normal value of BRS is about 15 ms/mmHg. The computation of BRS with the one or more PPG sensors 21 may be measured as a ratio of R-R interval variability over systolic arterial pressure.

Therefore, the one or more PPG sensors 21 may be configured to measure a pulse to pulse interval variability (PPV) as a substitute for R-R interval variability (RRV). The one or more PPG sensors 21 may be configured to measure a pulse parameter (PPar) indicative of blood pressure variability. The pulse parameter indicative of blood pressure variability may be a PPar indicative of systolic arterial pressure variability (SAPV). The processor 3 is configured to compute, based on the PPV and the pulse parameter indicative of the blood pressure variability, the BRS. The processor 3 may be configured to compute the BRS by dividing the PPV by the pulse parameter indicative of the blood pressure variability. The pulse parameter indicative of the blood pressure variability may correspond to the pulse up slope (PUS), the pulse amplitude (PA), the pulse width (PW) and/or the pulse slope transient time (PSTT). The aforementioned examples of a pulse parameter indicative of the blood pressure variability correspond to morphological features measureable by the one or more PPG sensors 21. They may be measured as a substitute for estimating systolic arterial pressure variability (SAPV).

The cerebral autoregulation (CAR) is the mechanism ensuring the maintenance of constant cerebral blood flow (CBF) over a specific range of systemic hemodynamic changes.

Near-infrared spectroscopy (NIRS) allows continuous, non-invasive, real-time monitoring of cerebral oxygen saturation (COS) in a small sample of the frontal cortex.

CAR may be computed as correlation between mean arterial blood pressure (MAP) and cerebral oxygen saturation (COS) measured by near-infrared spectroscopy (NIRS). NIRS-measured COS is considered a reliable surrogate of CBF. The cerebral oximetry index (Cox) may be the determining index for impaired CAR which may be calculated by correlation between COS and MAP. A value of Cox that is smaller than 0.5 and greater than 0.3 (0.5> Cox > 0.3) may be an indicator of impaired CAR. Impaired CAR is observed in patients with traumatic brain injury, ischemic strokes, and cerebral vascular stenosis. Assessment of CAR during recanalization therapy for acute ischemic stroke is relevant, and may influence future strategies for personalized blood pressure control and associated neuroprotection.

To assess CAR, changes in CBF may be plotted over a wide range of blood pressures. From a practical point of view, this may be done by a continuous measurement of mean arterial blood pressure (MAP) and a real-time estimate of CBF. Theoretically cerebral perfusion pressure (CPP) should be plotted. However, this requires invasive monitoring of intracranial pressure (ICP). The CPP is equal to the ICP subtracted from the MAP. Therefore, without access to ICP (because this requires invasive monitoring, whereas the system 1 is directed to mere non-invasive measurements), MAP is considered a valid substitute for CPP.

Therefore, the two or more NIRS sensors 22 may be configured to measure COS and MAP. The processor 3 is configured to compute, based on the COS and the MAP, the Cox. The processor may be configured to compute the Cox by computing the correlation between the COS and the MAP. Thus, the features measurable by the NIRS sensors may be extracted for quantification of CAR followed by computation of the Cox.

The cerebral blood flow reduction is also accompanied by changes in the pattern of brain electrical activities recorded on EEG. Hence brain function deteriorates and EEG signal patterns attenuate starting with the loss of fast gamma (30-80 Hz) waves, beta waves (12-20 Hz) and alpha waves (8-12 Hz), followed by dominance of theta (4-8 Hz) rhythm and finally slowing down to delta waves (0.5-4 Hz). Additionally interhemispheric spectral asymmetry of EEG rhythms may assist in stroke detection. Most strokes are unilateral, and thus deficits (e.g., motor impairments) are lateralized as well. Therefore, brain symmetry is another quantitative EEG measure that may been used to distinguish stroke from control populations.

Therefore, the processor 3 is configured to compute, based on results of measurements performed by the five or more EEG sensors 23, the EEG score being an indicator for electrical brain activity.

The five or more EEG sensors 23 may be configured to measure an electric power of alpha waves, an electric power of beta waves, an electric power of theta waves and/or an electric power of delta waves. In other words, the five or more EEG sensors may be configured to measure an electric power in the alpha frequency band, the beta frequency band, the theta frequency band and the delta frequency band.

The processor 3 may be configured to compute, based on the results of the measurements performed by the five or more EEG sensors 23, a ratio between a measured power of delta waves and a measured power of alpha waves (DAR). In addition or alternatively, the processor 3 may be configured to compute, based on the measurement results of the EEG sensors 23, a ratio between a sum of the measured power of delta waves and a measured power of theta waves and a sum of the measured power of alpha waves and a measured power of beta waves (DBATR). In addition or alternatively, the processor 3 may be configured to compute, based on the measurement results of the EEG sensors 23, a pairwise-derived brain symmetry index (PDBSI). A PDBSI greater than 0.3 (PDBSI > 0.3) indicates an impaired symmetry. When cerebral blood flow (CBF) drops during ischemia stroke, abnormalities, such as decreases, in fast frequencies (alpha waves, beta waves, theta waves and/or delta waves) may be observed. Abnormalities due to stroke, include increased DAR or DTABR ratio and changes in brain symmetry. This in particular occurs in the acute phase of stroke.

The processor 3 may be configured to compute, based on the DAR, DBATR and/or PDBSI, the EEG score. In particular, the processor 3 may be configured to compute based on the DAR, DBATR and PDBSI, the EEG score.

Due to activity of the person, the measurements results of the plurality of sensors 2 (in particular of the at least one PPG sensor 21, the NIRS sensors 22 and the EEG sensors 23) may be disturbed by the activity such that they are invalid. That is, they are not useable for a processing by the processor 3 for determining the risk of stroke of the person.

Therefore, the plurality of sensors 2 may optionally comprise the one or more inertial sensors 24. The processor 3 may be configured to detect, based on results of measurements performed by the one or more inertial sensors 24, activity of the person. The processor 3 may be configured to discard the obtained first parameter P1, second parameter P2 and cerebral electric activity parameter P3, in case of detecting activity of the person. The one or more inertial sensors 24 may optionally be used for checking balance of the person and detect stroke during a standing position of the person. For example, in case of the person falling to the bottom because of a stroke (i.e. the person losses his balance, this may be detected by the one or more inertial sensors 24 as an indicator of a stroke event).

As shown in Figure 2, the processor 3 may optionally be configured to obtain a patient clinical score 4 of the person. Optionally, the processor 3 may be configured to determine the risk of stroke of the person based on the patient clinical score 4 of the person in addition to the first parameter P1, the second parameter P2 and the cerebral electric activity parameter P3. According to an embodiment, the clinical stroke score 4 of the patient may be available via an application on the patient's smartphone. The patient clinical score 4 may be used to improve the accuracy of stroke risk determination by the system 1.

Further, the system 1 may optionally comprise a user interface 5 and/or a warning device 6. The user interface 5 and the warning device 6 may optionally correspond to the same device (not shown in Figure 2).

The user interface 5 is configured to inform the person on the determined risk of stroke (determined by the processor 3). In addition or alternatively, the user interface 5 is configured to allow the person to correct computation results of the processor 3. In addition or alternatively, the user interface 5 is configured to allow the person to input ground truth information with respect to a risk of stroke. The warning device 6 is configured to warn the person in case the determined risk of stroke is greater than a threshold for the risk.

The processor 3 is configured to communicate with the plurality of sensors (in particular the at least one PPG sensor 21, the NIRS sensors 22, the EEG sensors 23 and the optional at least one inertial sensor 24), the optional user interface 5 and the optional warning device 6. In particular, the processor 3 may receive data (measurement results) from the plurality of sensors and provide data (e.g. computation results) to the optional user interface 5 and the optional warning device 6. Optionally, the processor 3 is configured to communicate with extern, in particular for receiving the optional patient clinical score 4 of the person. The aforementioned communication may be wireless and/or wire bound according to any means known in the art (e.g. using Bluetooth, W-LAN etc. for a wireless communication method).

At least one sensor of the plurality of sensors 2, the processor 3, the optional user interface 5 and/or the optional warning device 6 may form or be part of a common device, in particular a common mobile device, optionally with a common housing. For example, the at least one PPG sensor 21, the at least one optional inertial sensor 24, the processor 3, the user interface 5 and the warning device 6 may be part of a watch, in particular smart watch, wearable by the person. The other sensors, that is the NIRS sensors 22 and the EEG sensors 23 may be part of a piece of clothing wearable at the head.

Alternatively, at least one of the at least one optional inertial sensor 24, the processor 3, the user interface 5 and the warning device 6 may be also part of the piece of clothing wearable at the head. For example, in case the piece of clothing wearable at the head correspond to glasses (e.g. smart glasses), the glasses may comprise besides the NIRS sensors 22 and the EEG sensors 23 the user interface 5 and the warning device 6. The user interface 5 may comprise or correspond to at least one display (e.g. transparent display) integrated in at least a part of the glasses that is arranged in front of the eyes of the person, when the person wears the glasses. The optional warning device 6 may comprise or correspond to at least one means for generating an audio stimulus (e.g. at least one loudspeaker), at least one means for generating a tactile stimulus (e.g. at least one vibrator) and/or the aforementioned at least one display.

According to a further embodiment, the sensors may be part of one or more sensor modules wearable by the person, e.g. a piece of clothing wearable at the head and optionally a smart watch. The processor 3, the optional user interface 5 and the optional warning device 6 may be part of a further device, such as a mobile device (e.g. portable phone).

For further details of the system 1 of Figure 2 reference is made to the above description of the system according to the first aspect of the present disclosure.

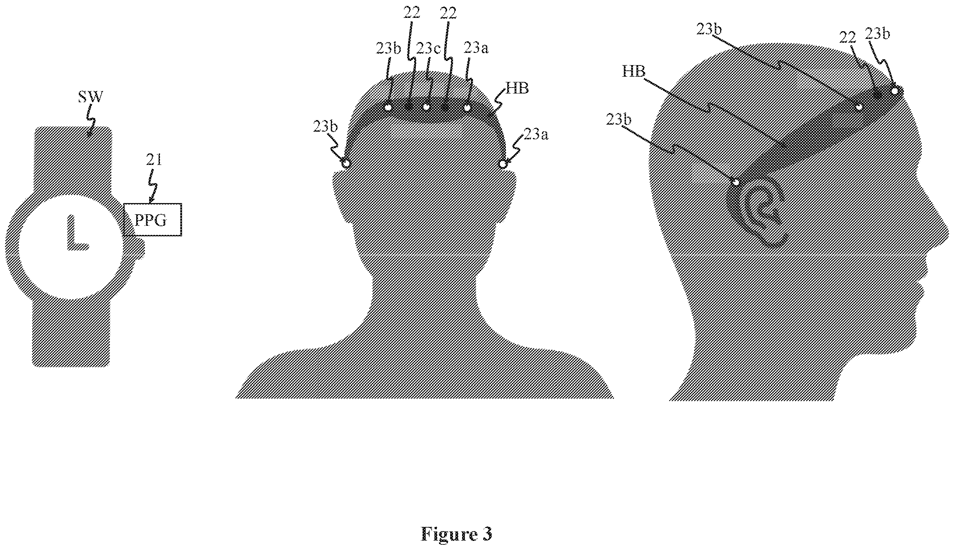

**Figure 3** shows an example of the arrangement of the plurality of sensors of a system according to an embodiment of the invention, when a person wears the plurality of sensors. The system of Figure 3 corresponds to the system 1 of Figure 2. Therefore, the description of the system 1 of Figures 1 and 2 is correspondingly valid for the system of Figure 3. In the following, mainly the arrangement of the plurality of sensors of the system is described.

As shown in Figure 3, the at least one PPG sensor 21 may be part of a watch SW, such as a smart watch. The watch SW is only one example of a mobile device wearable by the person, e.g. at the wrist, for exemplarily describing the arrangement of the plurality of sensors.

Therefore, the present disclosure is not limited thereto. The PPG sensor 21 may be part of any other mobile device wearable by the person. For example, the PPG sensor 21 may be part of a wristband, a breast band (wearable at the breast of the person) etc.

The at least two NIRS sensors 22 and the at least five EEG sensors 23 may be part of a headband HB wearable at the head. The headband HB is only one example of a piece of clothing wearable at the head that is used for exemplarily describing the arrangement of the plurality of sensors. Therefore, the present disclosure is not limited thereto. The at least two NIRS sensors 22 and the at least five EEG sensors 23 may be part of any other piece of clothing wearable at the head. For example, the piece of clothing wearable at the head may alternatively be a hat, a cap, glasses (e.g. smart glasses), a facial mask (e.g. sleeping mask, respiratory mask) etc.

The following description is with regard to the watch SW and the headband HB and is valid in case other elements wearable by the person are used.

Optionally, the watch SW may comprise at least one of the processor 3, the at least one optional inertial sensor 24, the optional user interface 5 and the optional warning device 6 (not shown in Figure 3). The headband HB optionally comprises the other element(s) of the processor 3, the at least one optional inertial sensor 24, the optional user interface 5 and the optional warning device 6 that are not comprised by the watch SW.

As indicated in Figure 3, the headband HB comprises the at least five EEG sensors 24 such that four EEG sensors 24 of the five EEG sensors 24 form two EEG sensor pairs each comprising a respective first EEG sensor 2a for measuring cerebral electric activity in the left cerebral hemisphere of the person and respective second EEG sensor 23b for measuring cerebral electric activity in the right cerebral hemisphere of the person. Thus, the headband HB comprises the at least five EEG sensors 24 such that the respective first EEG sensor 23a and the respective second EEG sensor 23b of each EEG sensor pair are positioned at corresponding positions of the two cerebral hemispheres of the person, when the person wears the headband HB. The fifth EEG sensor 23c may be used as a reference sensor (cf. the EEG sensor 23c positioned in the middle of the headband HB at the forehead shown in Figure 3). The at least two NIRS sensors 22 may be placed on the forehead, as shown in Figure 3. Three EEG sensors of the at least five EEG sensors 23 are configured to be positioned on the forehead and two EEG sensors of the at least five EEG sensors 23 are configured to be positioned at the head behind the ears. Optionally, the headband HB may comprise at least two additional EEG sensors that are configured to be positioned at an occipital position of the head, when the person wears the headband HB. The EEG sensors may comprise or correspond to electrodes that may be placed at the head of the person.

The watch SW and the headband may be configured to communicate with each other via Bluetooth. The watch SW and the headband HB may be configured to communicate to a smartphone (patient's smartphone) via Bluetooth. The smart phone may comprise the processor 3 and optionally the optional user interface 5 and/or the optional warning device 6.

For further details of the system 1 of Figure 3, in particular an arrangement of the plurality of sensors, reference is made to the above description of the system according to the first aspect of the present disclosure.

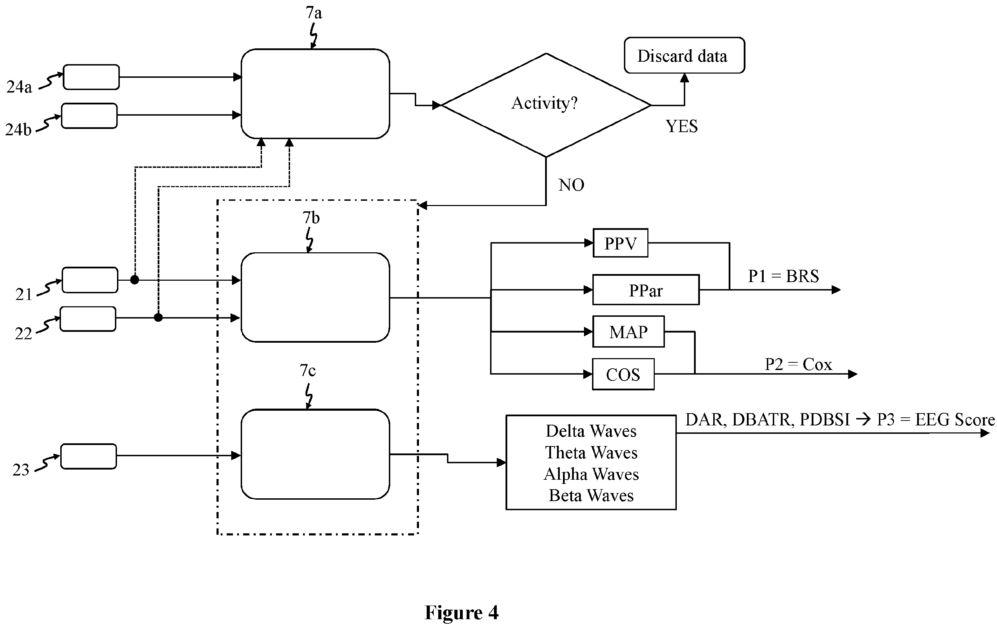

**Figure 4** shows an example of processing the results of measurements performed by the plurality of sensors of a system according to an embodiment of the invention. The system of Figure 4 may correspond to the system of any one of Figures 1 to 3. Therefore, the description of the system 1 of Figures 1 to 3 is correspondingly valid for the system of Figure 4.

According to Figure 4, the system comprises two optional inertial sensors, namely at least one accelerometer 24a and at least one angular rate sensor 24b (e.g. at least one gyroscope). This is only by way of example and, thus, the present disclosure is not limited thereto. The measurement results of the plurality of sensors may be processed before obtaining the first parameter P1, the second parameter P2 and the cerebral electric activity parameter P3 of the person. The processing of the results of measurements performed by a sensor may be performed by the sensor and/or the processor 3.

The processing 7a of the results of measurements performed by the at least one accelerometer 24a and at least one angular rate sensor 24b may comprise a feature extraction, a classification and an activity identification. As shown in Figure 4, based on the measurement results of the at least one accelerometer 24a and at least one angular rate sensor 24b, activity of the person may be detected (when the person wears these sensors). In case an activity is detected, the measurement results of the other sensors 21, 22 and 23 of the plurality of sensors of the system 1 are discarded. Alternatively, in case an activity is detected, the first parameter P1, the second parameter P2 and the cerebral electric activity parameter P3 of the person are discarded. In case no activity is detected the measurement results of the other sensors 21, 22 and 23 of the plurality of sensors may be used for obtaining the first parameter P1, the second parameter P2 and the cerebral electric activity parameter P3 of the person. As indicated in Figure 4 by dashed arrows, optionally, the detection of activity may be detected, based on the measurement results of the at least PPG sensor 21 and/or the at least two NIRs sensors 22 in addition to the measurement results of the at least one inertial sensor (which optionally corresponds to the at least one accelerometer 24a and the at least one angular rate sensor 24b as shown in Figure 4).

The processing 7b of the results of measurements performed by the at least one PPG sensor 21 and the at least two NIRS sensors 22 may comprise a filtering, a baseline correction and a feature extraction. The processing 7c of the results of measurements performed by the at least five EEG sensors 23 may comprise an artefact removal, a filtering, a time-frequency processing and a feature extraction. The measurement results of any one of the plurality of sensors of the system may alternatively or additionally be processed according to any signal processing respectively pre-processing (e.g. filtering, baseline correction, artefact removal, feature extraction etc.) known in the art.

As indicated in Figure 4, the at least one PPG sensor 21 is configured to provide the PPV and a pulse parameter (PPar) indicative of blood pressure variability, such as systolic arterial pressure variability (SAPV). The processor 3 is configured to compute, based on the PPV and the pulse parameter (PPar) the BRS as the first parameter P1. The at least two NIRS sensors 22 are configured to provide the MAP and COS and the processor 3 is configured to compute, based on the MAP and the COS, the Cox as the second parameter P2. The at least five EEG sensors 23 are configured to provide the measured electric alpha waves, electric beta waves, electric theta waves and delta waves. The processor 3 is configured to compute based on measured electric alpha waves, electric beta waves, electric theta waves and delta waves the DAR, DBATR and the PDBSI. The processor 3 is configured to compute based on the DAR, DBATR and the PDBSI the EEG score as the cerebral electric activity parameter P3.

In particular, the processor may be configured to compute the EEG score by combining the DAR, the DBATR and the PDBSI using a classifier. The classifier may be a tree baggers classifier. The processor 3 may be configured to input the DAR, the DBATR and the PDBSI to the tree baggers classifier that generates, based on the DAR, the DBATR and the PDBSI, classification trees. The processor may be configured to combine the generated classification trees to generate a final classification tree for generalizing the DAR, the DBATR and the PDBSI to the EEG score.

Thus, as described with regard to Figure 4, after pre-processing the measurement results of the plurality of sensors of the system, the relevant features with regard to determining the first parameter P1, the second parameter P2 and the cerebral electric activity parameter P3 may be extracted from each sensor/sensor combination.

For further details of the system of Figure 4, in particular processing the results of measurements performed by the plurality of sensors, reference is made to the above description of the system according to the first aspect of the present disclosure.

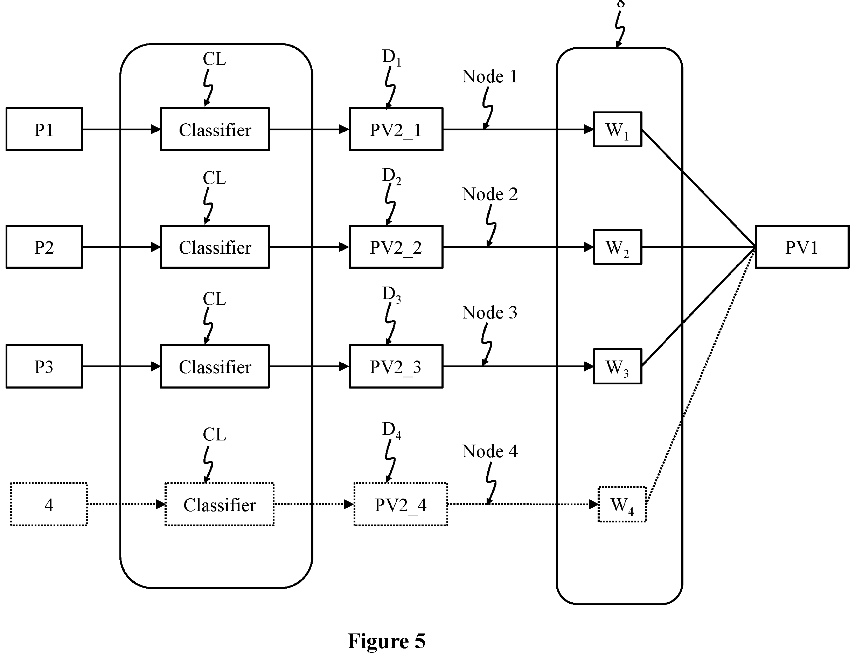

**Figure 5** shows an example of determining the risk of stroke of a person by computing a first probability vector by a processor of a system according to an embodiment of the invention. The system of Figure 5 may correspond to the system of any one of Figures 1 to 4. Therefore, the description of the system of Figures 1 to 4 is correspondingly valid for the system of Figure 5.

As indicated in Figure 5, the processor 3 of the system 1 may use the first parameter P1 dependent on blood pressure of the person, the second parameter P2 dependent on cerebral blood oxygenation of the person and the cerebral electric activity parameter P3 of the person for computing a first probability vector PV1 that is indicative of the risk of stroke of the person.

For this, the processor 3 is configured to perform the following computation: For each parameter of the first parameter P1, second parameter P2 and the cerebral electric activity parameter P3, the processor 3 computes, based on the parameter, a respective second probability vector PV2_1, PV2_2 respectively PV2_3 using a respective classifier CL. That is, based on the first parameter P1 the second probability vector PV2_1; based on the second parameter P2 the second probability vector PV2_2 and based on the cerebral electric activity parameter P3 the second probability vector PV2_3 is computed using the respective classifier CL. Since each second probability vector is an output of the respective classifier, each second probability vector correspond to a decision $D_1$, $D_2$ respectively $D_3$ of the respective classifier CL. Thus, the processor 3 is configured to compute, based on the aforementioned three parameters, the three second probability vectors PV2_1, PV2_2 and PV2_3 (using for each parameter the respective classifier CL).

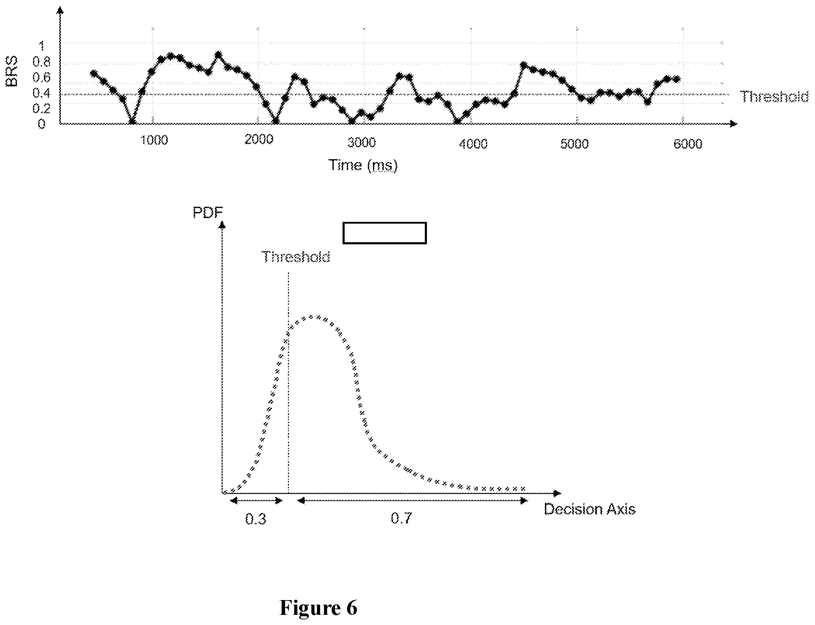

Each second probability vector indicates a risk of stroke of the person. The risk of stroke indicated by the second probability vector PV2_1 is computed based on the first parameter P1, the risk of stroke indicated by the second probability vector PV2_2 is computed based on the second parameter P2, and the risk of stroke indicated by the second probability vector PV2_3 is computed based on the cerebral electric activity parameter P3. The classifiers CL may correspond to a decision tree classifier, a support vector machine (SVM) classifier, k-nearest neighbors algorithm (KNN) classifier or any other known classifier type. Optionally, the classifiers CL are of the same classifier type. Alternatively, at least one of the classifiers CL may be of a different classifier type than the other. An example of how a classifier CL is configured to compute a decision (e.g. $D_1$) in the form of a second probability vector (e.g. PV2_1) is shown in Figure 6 for the first parameter P1 corresponding to the BRS.

As shown in Figure 5, the processor 3 is configured to compute the first probability vector PV1 by combining the three second probability vectors PV2_1, PV2_2 and PV2_3 using a weighted sum. In particular, the processor is configured to compute the first probability vector PV1 by using a trained machine learning model 8 that weights each of the three second probability vectors PV2_1, PV2_2 and PV2_3 with a respective weight $W_1$, $W_2$ respectively $W_3$ and sums the three weighted second probability vectors to generate the first probability vector PV1. The machine learning model 8 (in particular the weights $W_1$, $W_2$ and $W_3$) is trained using training data comprising a plurality of data sets. Each data set comprises for a respective time segment one or more samples of the first parameter P1, the second parameter P2 and the cerebral electric activity parameter P3 associated with a probability vector indicating at least a known risk of stroke for the respective time segment.

During training, the three parameters P1, P2 and P3 of a data set of the training data are used for computing three second probability vectors PV2_1, PV2_2 and PV2_3, as described above, which are input into the machine learning model 8 generating a first probability vector PV1 for the data set. The generated first probability vector PV1 is compared with the probability vector (ground truth) associated with the three parameters P1, P2 and P3 of the data set to compute a difference (error) between the probability vector of the data set and the generated first probability vector PV1. Based on the computed difference (error) the weights $W_1$, $W_2$ and $W_3$ of the machine learning model are adjusted respectively updated to minimize the difference (error). The aforementioned is repeated with further data sets of the training data until the computed difference (error) is smaller than a threshold value.

In particular, a plurality of the above described data sets (input-output data pairs) may be obtained. Each data set comprises for a respective time segment one or more samples of the first parameter P1, the second parameter P2 and the cerebral electric activity parameter P3 associated with a probability vector indicating at least a known risk of stroke for the respective time segment. For example, each data set may comprise annotated data where stroke instances occurred during the respective time segment are marked (e.g. by medical doctors). The plurality of data sets may be divided into training data and a validation/test set. For example, the plurality of data sets may be split into 90% for training and 10% for testing. Using the very 10 cross-validation, repeatedly nine out of these ten portions may be used as training data to train the machine learning model 8 and, thus, generate the trained machine learning model 8, while the tenth may be used as test data. The performance of the classifiers CL may be evaluated by using performance indices such as sensitivity, specificity, accuracy and F measure (F score). Each data set may comprise hours of annotated data labeled with ground truth, and the three parameters P1, P2 and P3 (which may correspond or be indicative for the biomarkers: BRS, CAR and EEG).

The probability vectors PV1, PV2_1, PV2_2 and PV2_3 may comprises at least two entries indicating at least two probabilities of at least two classes with regard to risk of stroke. For example, the probability vectors PV1, PV2_1, PV2_2 and PV2_3 may comprises two entries, wherein a first entry of the two entries indicates the probability of no risk of stroke and a second entry of the two entries indicates the probability of a risk of stroke. In this case, the two classes correspond to "no risk of stroke" and "risk of stroke". This is only by way of example and does not limit the present disclosure, because the probability vectors may have more than two entries.

The processor 3 may be configured to performs risk of stroke determination based on N parameters obtained based on measurement results of N sensor nodes $S = \{s_i \mid i = 1,2,...N\}$. When using the three parameters P1, P2 and P3, N is equal to three (N = 3), as it is the case in Figure 5. The N sensor nodes collect information on events taking place within their sensing ranges. The processor is configured to classify, based on the three parameters P1, P2 and P3, these events into predefined classes $E = \{e_j \mid j = 1,2,... t\}$, wherein t is the maximum number of classes. For example, t may be equal to two classes(t = 2): first class e1 may indicate no risk of stroke and second class e2 may indicated risk of stroke.

For example, with respect to Figure 5, the second probability vector PV2_1 (corresponding to decision $D_1$) may equal to (0.7, 0.3), wherein the first entry indicates the class "no risk of stroke" and the second entry indicates the class "risk of stroke". This implies that node 1 classifies, based on the first parameter P1, the event as no risk of stroke with a probability of 70% and the event as risk of stroke with a probability of 30%.

The first probability vector PV1 (corresponding to a final decision) for an event $e_i$ may be denoted as $X_i = (x_{i1}, x_{i2}, ....,x_{im})$, wherein m correspond to the number of possible classes for event $e_i$. A decision $D_i$ (in the form of a second probability vector) for an event $e_i$ may be denoted as $D_i = (d_{i1}, d_{i2}, ..., d_{im})$. As mentioned above, it is assumed that m = 2. Each sensor node is associated with a weight $W_j$. A sensor node with higher weight means that the node has more decision making power, i.e. has greater influence on the entries of the first probability vector PV1.

For optimizing the process performed by the processor based on the three parameters P1, P2 and P3 using the classifiers CL and the machine learning model 8, an optimization problem P may be solved by:

$$A = (a_{ij})_{t*N}$$

In the above, $a_{ij}$ indicates whether event $e_i$ is observed by sensor node $s_j$

The optimization problem P may be solved by minimizing the disagreement between the weighted summation of distances between the decisions and individual sensor nodes D and decision X. For this a L2 norm may be used as a distance function.

$$\| X_i - D_i \|^2 = (x_{i1} - d_{i1})^2 + (x_{i2} - d_{i2})^2$$

$$P: \min \sum_{i=1}^{t} \sum_{j=1}^{N} a_{ij} w_j \|X_i - D_i^j\|^2$$

The initial final decision X may be generated by either averages of individual sensor nodes or majority voting of the decisions $D_1$, $D_2$ and $D_3$. In each iteration of the optimization, the values of the weights $W_1$, $W_2$ and $W_3$ and aggregated decision X (i.e. the first probability vector PV1) are updated alternatively and separately. In particular, in a first step, the weight $W_1$, $W_2$, $W_3$ of each node (Node 1, Node 2 and Node 3) is fixed, and the optimization problem P is initially computed with respect to the aggregated decision ($X_i$). In a second step, the computed decision X is fixed and the optimization problem P is solved with respect to the weights $W_1$, $W_2$, $W_3$. This two-step process is repeated until convergence occurs.

As indicated in Figure 5 by the dashed lines, optionally, the processor 3 may use, in addition to the first parameter P1, the second parameter P2 and the cerebral electric activity parameter P3, a patient clinical score 4 of the person, for computing the first probability vector PV1.

The above description is correspondingly valid for this case, wherein the decision $D_4$ is computed by the classifier CL based on the patient clinical score 4 and output in the form of the second probability vector PV2_4. The decision $D_4$ is assumed to be associated to a further sensor node "Node 4".

For further details of the system of Figure 5, in particular the computation of the first probability vector, reference is made to the above description of the system according to the first aspect of the present disclosure.

**Figure 6** shows an example of processing, using a classifier, baroreflex sensitivity (BRS) by a processor of a system according to an embodiment of the invention. The system of Figure 6 may correspond to the system of any one of Figures 1 to 5. Therefore, the description of the system 1 of Figures 1 to 5 is correspondingly valid for the system of Figure 6.

For the following description, it is exemplarily assumed that each probability vector comprises two entries indicating two probabilities of two classes with regard to risk of stroke. For example the two classes may correspond to "no risk of stroke" and "risk of stroke".

The processor 3 may be configured to obtain, based on the results of the measurements performed by the plurality of sensors, a time series of each parameter of the first parameter P1, the second parameter P2 and the cerebral electric activity parameter P3. The processor may be configured to process the parameter by sampling for a time segment (time window) the time series of the parameter and comparing each sample with at least one threshold for the parameter.

In particular, the processor may use a moving time window for processing a time series of a parameter by a classifier. The processor is configured to make a decision for a selected time segment (time window) in the time series using the classifier. For example, in case the time segment equals to 60 seconds and the sampling rate Fs is equals to one hundred hertz (Fs = 100 Hz), then in each time segment (time window) 60*100= 6000 data points are sampled for a respective parameter, as indicated in Figure 6 for the first parameter P1 equaling to the BRS.

A value of the BRS smaller than 3 ms/mmHG (BRS< 3 ms/mmHG) may indicate a risk of stroke, because hypertensive stroke patients with metabolic syndrome have critical BRS values smaller than 3 ms/mmHg. Therefore, the BRS value of 3 ms/mmHg may be used as a threshold value for classification using a probability density function (PDF) for processing the first parameter P1 by the respective classifier CL.

For the classification, the first input parameter P1, i.e. the BRS, and the corresponding threshold value may be normalized to be between [0 1] using the following formula

$$z_i = \frac{x_i - \min(x)}{\max(x) - \min(x)}$$

For the first parameter P1 (equaling to the BRS), the respective classifier CL may be configured to compute for the time segment the at least two probabilities indicated by the at least two entries of the respective second probability vector PV2_1 by comparing each sample of the BRS (of the time segment) with the threshold of 3 ms/mmHg. That is, the threshold may be applied on the probability density function (PDF) to calculate the probability for each class (e.g. no risk, risk) as shown at the bottom of Figure 6. In the case of Figure 6, the second probability vector PV2_1 corresponding to the decision $D_1$ computed by the respective classifier CL based on the first parameter P1 is assumed to be (0.7, 0.3). This may indicate a probability of 70% of no risk of stroke and a probability of 30% of a risk of stroke.

In case the second parameter P2 corresponds to the Cox, a value of the Cox smaller than 0.5 and greater than 0.3 (0.5 > Cox > 0.3) may indicate a risk of stroke, because a value of Cox that is smaller than 0.5 and greater than 0.3 may be an indicator of impaired CAR. Therefore, the Cox values of 0.3 and 0.5 may be used as threshold values for classification using a probability density function (PDF) for processing the second parameter P2 by the respective classifier CL.

In case the cerebral electric activity parameter P3 corresponds to the EEG score corresponding to the PDBSI, a value of the PDBSI greater than 0.3 (PDBSI > 0.3) may indicate a risk of stroke, because a value of the PDBSI greater than 0.3 indicates an impaired symmetry. Therefore, the PDBSI value of 0.3 may be used as a threshold value for classification using a probability density function (PDF) for processing the cerebral electric activity parameter P3 by the respective classifier CL.

For the classification, the second input parameter P2 respectively the cerebral electric activity parameter P3, and the corresponding threshold value(s) may be normalized to be between [0 1].

The above description of classification based on the first parameter P1 is correspondingly valid for a classification based on the second input parameter P2 and the cerebral electric activity parameter P3. Thus, for each parameter P1, P2 and P3 the respective decision $D_1$, $D_2$ respectively $D_3$ may be obtained as shown in Figure 6.

For further details of the system of Figure 6, in particular processing the BRS, reference is made to the above description of the system according to the first aspect of the present disclosure.

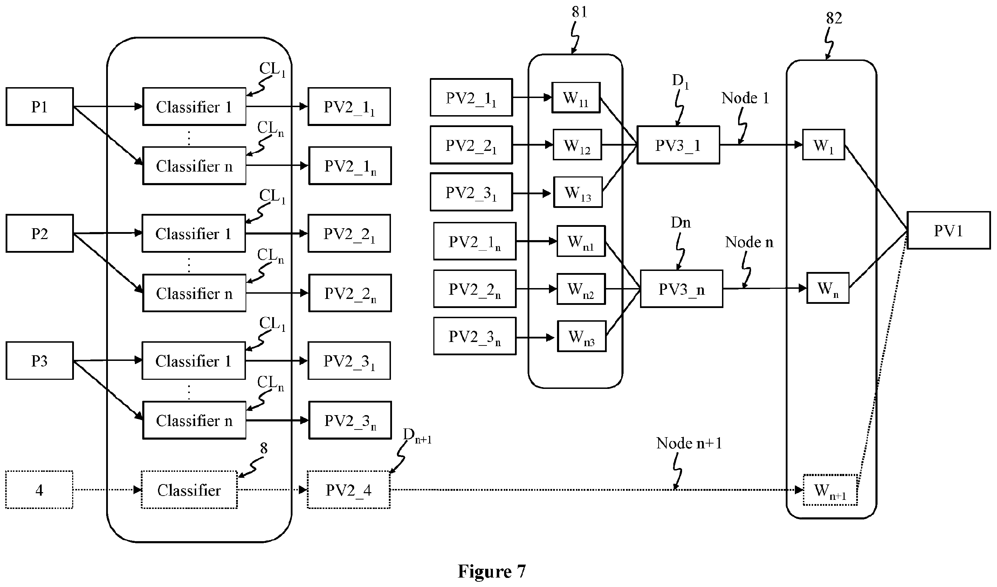

**Figure 7** shows an example of determining the risk of stroke of a person by computing a first probability vector by a processor of a system according to an embodiment of the invention. The system of Figure 7 may correspond to the system of any one of Figures 1 to 6. Therefore, the description of the system of Figures 1 to 6 is correspondingly valid for the system of Figure 7.

As shown in Figure 7, the processor 3 may use n classifiers for processing each parameter of the first parameter P1, the second parameter P2 and the cerebral electric activity parameter P3, wherein n is greater or equal to two ($n \geq 2$, 3, 4, ...). This generates for each parameter n second probability vectors, e.g. for the first parameter P1 the n second probability vectors PV2_$1_1$, ..., PV2_$1_n$. Thus, according to the embodiment of Figure 7 the processor 3 may combine a multi-sensor model (at least the three input parameters P1, P2 and P3) and a multi-classifier model (multiple classifiers $CL_1$,..., $CL_n$) to form a hierarchical fusion model (multi-sensor multi-classifier hierarchical fusion model) to predict the class label of an event. Combining multiple classifiers for decision making has a great effect on improving the recognition performance. In classifier fusion, multiple basic classifiers types may be used (e.g. decision tree, SVM, KNN etc.), to evaluate classifier performance for fusion algorithm. The n classifier types may correspond to any classifiers known (e.g. decision tree, SVM, KNN etc.).

As shown in Figure 7, for each classifier type of the n classifier types $CL_1$, ..., $CL_n$, the processor 3 may be configured to compute a respective third probability vector by combining the three respective second probability vectors, computed by the classifier type, using a weighted sum. For this, a first trained machine learning model 81 may be used. As a result, the processor may be configured to compute n third probability vectors $PV3\_1$, ..., $PV3\_n$. For example, for the classifier type $CL_1$, the processor is configured to compute the third probability vector $PV3\_1$ by combining the three second probability vectors $PV2\_1_1$, $PV2\_2_1$ and $PV2\_3_1$ that have been computed by the classifier type $CL_1$. The processor 3 may be configured to compute the first probability vector PV1 by combining the n third probability vectors $PV3\_1$, ..., $PV3\_n$ using a weighted sum. For this, a second trained machine learning model 82 may be used.

For describing the training of the processing (performable by the processor 3) shown in Figure 7, in particular training the first machine learning model 81 and second machine learning model 82, reference is made to the corresponding description with respect to training of the embodiment according to Figure 5.

In a first step of a training phase, the n decisions $D_1$....$D_n$ may be obtained by using majority voting. Each decision is a third probability vector representing the classification performance. Each classifier $CL_1$, ..., $CL_n$ has equal opportunity for majority voting. Hence, a strong classifier type may be highlighted for a real life implementation. Majority voting is a data fusion algorithm at the decision making level.

During a training of the processing (performable by the processor 3) shown in Figure 7, many decision results may be obtained using multiple classifiers $CL_1$ and $CL_n$. In the testing phase, the classifier that has outperform the rest may be used by adjusting the weighting of the first machine learning model 81, accordingly. That is, by adjusting the weights $W_{11}$, $W_{12}$, $W_{13}$, ..., $W_{n1}$, $W_{n2}$ and $W_{n3}$, accordingly.

A second step of the training phase is identical to the training explained with respect to machine learning model 8 shown in Figure 5.

The n decisions $D_1$, ..., $D_n$ are individual decisions for the n classifier types $CL_1$, ..., $CL_n$. Each probability vector may for example comprise four entries. A first entry of the four entries indicates the probability of a probability of stroke smaller than or equal to a first probability threshold. A second entry of the four entries indicates the probability of a probability of stroke greater than the first probability threshold and smaller than or equal to a second probability threshold. A third entry of the four entries indicates the probability of a probability of stroke greater than the second probability threshold. A fourth entry of the four entries indicates the probability that the first parameter, the second parameter and/or the cerebral electric activity parameter, used for determining the probability vector, were not suitable for determining the probability vector. Thus, the first entry indicates the probability of a low risk and the second entry indicates the probability of a medium risk. The third entry indicates the probability of a high risk and the fourth entry indicates the probability of no suitable data.

The first probability vector PV1 corresponds to an aggregated decision X. Each node "Node 1", ..., "Node n" is associated with a weight ($W_1$, ..., $W_N$), wherein higher weights indicate higher reliability. The aggregated decision X should favor more reliable nodes and weigh less unreliable ones.

The decision X may combine individual decisions (e.g. $D_1 = (0.1, 0.7, 0.1, 0.1)$, $D_2 = (0.3, 0.4, 0.1, 0.2)$, when n = 2 and D = (low risk, medium risk, high risk, no suitable data)). The decision X may be computed based on majority voting. Then an optimization problem P based on coordinate decent may be performed as outlined above with respect to Figure 5, with the difference that according to the embodiment of Figure 7 four classes are considered ("low risk", "medium risk", "high risk" and "no suitable data"):

$$\| X_i\text{-}D_i \|^2 = (x_{i1}\text{-}d_{i1})^2 + (x_{i2}\text{-}d_{i2})^2 + (x_{i3}\text{-}d_{i3})^2 + (x_{i4}\text{-}d_{i4})^2$$

$$P: \min \sum_{i=1}^{t} \sum_{j=1}^{n} a_{ij} w_j \| X_i - D_i^j \|^2$$

Aij is an observation matrix, shown below:

|  | Node1 | Node2 | .... | Node n |  |
|---|---|---|---|---|---|
| Event 1 | A11 | A12 | .... | A1n | low risk |
| Event 2 | A21 | A22 | .... | A2n | medium risk |
| Event 3 | A31 | A32 | .... | A3n | high risk |
| Event 4 | A41 | A42 | .... | A4n | no suitable data |

The observation matrix Aij indicates whether event i is observed by Node j.

As indicated in Figure 7 by the dashed lines, optionally, the processor 3 may use, in addition to the first parameter P1, the second parameter P2 and the cerebral electric activity parameter P3, a patient clinical score 4 of the person, for computing the first probability vector PV1.

The above description is correspondingly valid for this case, wherein the decision $D_{n+1}$ is computed by the classifier CL based on the patient clinical score 4 and output in the form of the second probability vector PV2_4. The decision $D_{n+1}$ is assumed to be associated to a further sensor node "Node n+1".

For further details of the system of Figure 7, in particular the computation of the first probability vector, reference is made to the above description of the system according to the first aspect of the present disclosure.

The features of the embodiments of Figures 1 to 7 may be combined to provide a system according to the present disclosure.

As outlined above, the present disclosure provides an automated stroke detection system to accurately assess the onset and/or progression of an ischemia stroke with noninvasive, portable, low cost devices. The system allows preventing post-stroke patients from risk of mortality or long-term disability.

The present disclosure has been described in conjunction with various embodiments as examples as well as implementations. However, other variations can be understood and effected by those persons skilled in the art and practicing the claimed subject matter, from the studies of the drawings, this disclosure and the independent claims. In the claims as well as in the description the word "comprising" does not exclude other elements or steps and the indefinite article "a" or "an" does not exclude a plurality. A single element or other unit may fulfill the functions of several entities or items recited in the claims. The mere fact that certain measures are recited in the mutual different dependent claims does not indicate that a combination of these measures cannot be used in an advantageous implementation.

**Claims**

1. A system (1) for determining a risk of stroke for a person, the system (1) comprising

   - a plurality of sensors (2), wearable by the person, and a processor (3); wherein
   - the plurality of sensors (2) is configured to perform non-invasive measurements at the person for obtaining a first parameter (P1) dependent on blood pressure of the person, a second parameter (P2) dependent on cerebral blood oxygenation of the person, and a cerebral electric activity parameter (P3) of the person; and
   - the processor (3) is configured to

      - obtain, based on results of the measurements performed by the plurality of sensors (2), the first parameter (P1), the second parameter (P2) and the cerebral electric activity parameter (P3), and
      - determine the risk of stroke of the person based on the first parameter (P1), the second parameter (P2) and the cerebral electric activity parameter (P3).

2. The system (1) according to claim 1, wherein

   - the first parameter (P1) corresponds to the baroreflex sensitivity, BRS, of the person, and
   - the plurality of sensors (2) comprises at least one photoplethysmographic, PPG, sensor (21) configured to measure a pulse to pulse interval variability, PPV, and a pulse parameter indicative of blood pressure variability, optional systolic arterial pressure variability; wherein
   - the processor (3) is configured to compute, based on the PPV and the parameter indicative of the blood pressure variability, the BRS.

3. The system (1) according to claim 2, wherein the pulse parameter indicative of the blood pressure variability corresponds to at least one of:

   - pulse up slope, PUS;
   - pulse amplitude, PA;
   - pulse width, PW; and
   - pulse slope transient time, PSTT.

4. The system (1) according to any one of the previous claims, wherein

   - the second parameter (P2) corresponds to the cerebral oximetry index, and

- the plurality of sensors (2) comprises at least two near infrared spectroscopy, NIRS, sensors (22) configured to measure cerebral oxygen saturation, COS, and mean arterial blood pressure, MAP; wherein
- the processor (3) is configured to compute, based on the COS and the MAP, the cerebral oximetry index.

5. The system (1) according to any one of the previous claims, wherein

- the cerebral electric activity parameter (P3) corresponds to an encephalography, EEG, score,
- the plurality of sensors (2) comprises at least five EEG sensors (23),
- the processor (3) is configured to compute, based on results of measurements performed by the at least five EEG sensors (23), the EEG score.

6. The system (1) according to claim 5, wherein

- the processor (3) is configured to compute, based on the results of the measurements performed by the at least five EEG sensors (23), at least one of:

    - a ratio between a measured power of delta waves and a measured power of alpha waves, DAR,
    - a ratio between a sum of the measured power of delta waves and a measured power of theta waves and a sum of the measured power of alpha waves and a measured power of beta waves, DBATR, and
    - a pairwise-derived brain symmetry index, PDBSI; and

- the processor (3) is configured to compute, based on at least one of the DAR, DBATR and PDBSI, the EEG score.

7. The system (1) according to any one of the previous claims, wherein

- the plurality of sensors (2) comprises at least one inertial sensor (24, 24a, 24b), and
- the processor (3) is configured to detect, based on results of measurements performed by the at least one inertial sensor (24, 24a, 24b), activity of the person and discard the obtained first parameter (P1), second parameter (P2) and cerebral electric activity parameter (P3), in case of detecting activity of the person.

8. The system (1) according to any one of the previous claims, wherein the processor (3) is configured to

- obtain a patient clinical score (4) of the person, and
- determine the risk of stroke of the person based on the patient clinical score of the person (4) in addition to the first parameter (P1), the second parameter (P2) and the cerebral electric activity parameter (P3).

9. The system (1) according to any one of the previous claims, wherein

- the processor (3) is configured to compute, based on the first parameter (P1), the second parameter (P2) and the cerebral electric activity parameter (P3), a first probability vector (PV1) to determine the risk of stroke of the person, wherein

the first probability vector (PV1) comprises at least two entries indicating at least two probabilities of at least two classes with regard to risk of stroke.

10. The system (1) according to claim 9, wherein

- for each parameter of the first parameter (P1), the second parameter (P2) and the cerebral electric activity parameter (P3), the processor (3) is configured to compute, based on the parameter (P1; P2; P3), a respective second probability vector (PV2_1; PV2_2; PV2_3) using a respective classifier (CL), to compute three second probability vectors (PV2_1, PV2_2, PV2_3), wherein
each of the three second probability vectors (PV2_1, PV2_2, PV2_3) comprises at least two entries indicating the at least two probabilities of the at least two classes with regard to risk of stroke, and
- the processor (3) is configured to compute the first probability vector (PV1) by combining the three second probability vectors (PV2_1, PV2_2, PV2_3) using a weighted sum.

11. The system (1) according to claim 10, wherein

- the processor (3) is configured to compute the first probability vector (PV1) by using a trained machine learning model (8) that weights each of the three second probability vectors (PV2_1, PV2_2, PV2_3) and sums the three weighted second probability vectors to generate the first probability vector (PV1), wherein
- the machine learning model is trained using training data comprising a plurality of data sets, wherein each data set comprises

- for a respective time segment one or more samples of the first parameter (P1), the second parameter (P2) and the cerebral electric activity parameter (P3) associated with a probability vector indicating at least a known risk of stroke for the respective time segment.

12. The system (1) according to claim 9, wherein

- for each parameter of the first parameter (P1), the second parameter (P2) and the cerebral electric activity parameter (P3), the processor (3) is configured to compute, based on the parameter (P1; P2; P3), two or more respective second probability vectors ($PV2\_1_1$, $PV2\_1_n$; $PV2\_2_1$, $PV2\_2_n$; $PV2\_3_1$, $PV2\_3_n$) using two or more respective classifiers ($CL_1$, $CL_n$), wherein the two or more respective classifiers ($CL_1$, $CL_n$) correspond to two or more classifier types,
- for each classifier type of the two or more classifier types ($CL_1$, $CL_n$), the processor is configured to compute a respective third probability vector (PV3_1; PV3_n) by combining three respective second probability vectors ($PV2\_1_1$, $PV2\_2_1$, $PV2\_3_1$; $PV2\_1_n$, $PV2\_2_n$, $PV2\_3_n$), computed by the classifier type ($CL_1$; $CL_n$), using a weighted sum to compute two or more third probability vectors (PV3_1, PV3_n), and
- the processor (3) is configured to compute the first probability vector (PV1) by combining the two or more third probability vectors (PV3_1, PV3_n) using a weighted sum.

13. The system (1) according to claim 12, wherein

- the processor (1) is configured to compute the first probability vector (PV1) by using two trained machine learning models (81, 82), wherein

for each classifier type ($CL_1$; $CL_n$), a first trained machine learning model (81) of the two models (81, 82) weights each of the three respective second probability vectors ($PV2\_1_1$, $PV2\_2_1$, $PV2\_3_1$; $PV2\_1_n$, $PV2\_2_n$, $PV2\_3_n$) and sums the three weighted second probability vectors to generate the respective third probability vector (PV3_1; PV3_n),
a second trained machine learning model (82) of the two models (81, 82) weights each of the two or more third probability vectors (PV3_1, PV3_n) and sums the two or more weighted third probability vectors to generate the first probability vector (PV1), and

- the two machine learning models (81, 82) are trained using training data comprising a plurality of data sets, wherein each data set comprises

- for a respective time segment one or more samples of the first parameter (P1), the second parameter (P2) and the cerebral electric activity parameter (P3) associated with a probability vector indicating at least a known risk of stroke for the respective time segment.

14. The system (1) according to any one of claims 9 to 13, wherein

- each probability vector comprises two entries, wherein a first entry of the two entries indicates the probability of no risk of stroke and a second entry of the two entries indicates the probability of a risk of stroke;
- each probability vector comprises three entries, wherein a first entry of the three entries indicates the probability of a probability of stroke smaller than or equal to a first probability threshold, a second entry of the three entries indicates the probability of a probability of stroke greater than the first probability threshold and smaller than or equal to a second probability threshold and a third entry of the three entries indicates the probability of a probability of stroke greater than the second probability threshold; or
- each probability vector comprises four entries, wherein a first entry of the four entries indicates the probability of a probability of stroke smaller than or equal to a first probability threshold, a second entry of the four entries indicates the probability of a probability of stroke greater than the first probability threshold and smaller than or equal to a second probability threshold, a third entry of the four entries indicates the probability of a probability of stroke greater than the second probability threshold, and a fourth entry of the four entries indicates the probability that the first parameter, the second parameter and/or the cerebral electric activity parameter, used for determining the probability vector, were not suitable for determining the probability vector.

15. The system (1) according to any one of the previous claims, wherein

- the system (1) comprises a user interface (5) for

- informing the person on the determined risk of stroke,
- allowing the person to correct computation results of the processor, and/or
- allowing the person to input ground truth information with respect to a risk of stroke; and/or

- the system (1) comprises a warning device (6) configured to warn the person in case the determined risk of stroke is greater than a threshold for the risk.

**Patentansprüche**

1. System (1) zum Bestimmen eines Schlaganfallrisikos einer Person, wobei das System (1) Folgendes umfasst:

- eine Vielzahl von Sensoren (2), die durch die Person tragbar ist, und einen Prozessor (3); wobei
- die Vielzahl von Sensoren (2) dazu konfiguriert ist, nichtinvasive Messungen an der Person durchzuführen, um einen ersten Parameter (P1), der von einem Blutdruck der Person abhängig ist, einen zweiten Parameter (P2), der von einer zerebralen Blutsauerstoffsättigung der Person abhängig ist, und einen Parameter zerebraler elektrischer Aktivität (P3) der Person zu erlangen; und
- der Prozessor (3) zu Folgendem konfiguriert ist:

- Erlangen, basierend auf Ergebnissen der Messungen, die durch die Vielzahl von Sensoren (2) durchgeführt werden, des ersten Parameters (P1), des zweiten Parameters (P2) und des Parameters zerebraler elektrischer Aktivität (P3) und
- Bestimmen des Schlaganfallrisikos der Person basierend auf dem ersten Parameter (P1), dem zweiten Parameter (P2) und dem Parameter zerebraler elektrischer Aktivität (P3).

2. System (1) nach Anspruch 1, wobei

- der erste Parameter (P1) der Baroreflexempfindlichkeit, BRS, der Person entspricht und
- die Vielzahl von Sensoren (2) mindestens einen photoplethysmographischen Sensor, PPG-Sensor (21), umfasst, der dazu konfiguriert ist, eine Puls-zu-Puls-Intervallvariabilität, PPV, und einen Pulsparameter zu messen, der eine Blutdruckvariabilität, gegebenenfalls eine systolische arterielle Druckvariabilität, angibt; wobei
- der Prozessor (3) dazu konfiguriert ist, basierend auf der PPV und dem Parameter, der die Blutdruckvariabilität angibt, die BRS zu berechnen.

3. System (1) nach Anspruch 2, wobei der Pulsparameter, der die Blutdruckvariabilität angibt, mindestens einem von Folgendem entspricht:

- einem Pulsanstieg, PUS;
- einer Pulsamplitude, PA;
- einer Pulsbreite, PW; und
- einer Pulssteigungsübergangszeit, PSTT.

4. System (1) nach einem der vorhergehenden Ansprüche, wobei

- der zweite Parameter (P2) dem zerebralen Oximetrieindex entspricht und
- die Vielzahl von Sensoren (2) mindestens zwei Nahinfrarotspektroskopiesensoren, NIRS-Sensoren (22), umfasst, die dazu konfiguriert sind, eine zerebrale Sauerstoffsättigung, COS, und einen mittleren arteriellen Blutdruck, MAP, zu messen; wobei
- der Prozessor (3) dazu konfiguriert ist, basierend auf der COS und dem MAP den zerebralen Oximetrieindex zu berechnen.

**5.** System (1) nach einem der vorhergehenden Ansprüche, wobei

- der Parameter zerebraler elektrischer Aktivität (P3) einem Enzephalographiepunktwert, EEG-Punktwert, entspricht,
- die Vielzahl von Sensoren (2) mindestens fünf EEG-Sensoren (23) umfasst,
- der Prozessor (3) dazu konfiguriert ist, basierend auf Ergebnissen von Messungen, die durch die mindestens fünf EEG-Sensoren (23) durchgeführt werden, den EEG-Punktwert zu berechnen.

**6.** System (1) nach Anspruch 5, wobei der Prozessor (3) dazu konfiguriert ist, basierend auf den

- Ergebnissen der Messungen, die durch die mindestens fünf EEG-Sensoren (23) durchgeführt werden, mindestens eines von Folgendem zu berechnen:

  - einem Verhältnis zwischen einer gemessenen Leistung von Deltawellen und einer gemessenen Leistung von Alphawellen, DAR,
  - einem Verhältnis zwischen einer Summe der gemessenen Leistung von Deltawellen und einer gemessenen Leistung von Thetawellen und einer Summe der gemessenen Leistung von Alphawellen und einer gemessenen Leistung von Betawellen, DBATR, und
  - einem paarweise abgeleiteten Gehirnsymmetrieindex, PDBSI; und
- der Prozessor (3) dazu konfiguriert ist, basierend auf mindestens einem des DAR, des DBATR und des PDBSI den EEG-Punktwert zu berechnen.

**7.** System (1) nach einem der vorhergehenden Ansprüche, wobei

- die Vielzahl von Sensoren (2) mindestens einen Trägheitssensor (24, 24a, 24b) umfasst, und
- der Prozessor (3) dazu konfiguriert ist, basierend auf Ergebnissen von Messungen, die durch den mindestens einen Trägheitssensor (24, 24a, 24b) durchgeführt werden, eine Aktivität der Person zu detektieren und im Falle des Detektierens der Aktivität der Person, den erlangten ersten Parameter (P1), zweiten Parameter (P2) und Parameter zerebraler elektrischer Aktivität (P3) zu verwerfen.

**8.** System (1) nach einem der vorhergehenden Ansprüche, wobei der Prozessor (3) zu Folgendem konfiguriert ist:

- Erlangen eines klinischen Patientenpunktwerts (4) der Person und
- Bestimmen des Schlaganfallrisikos der Person basierend auf dem klinischen Patientenpunktwert der Person (4) zusätzlich zu dem ersten Parameter (P1), dem zweiten Parameter (P2) und dem Parameter zerebraler elektrischer Aktivität (P3).

**9.** System (1) nach einem der vorhergehenden Ansprüche, wobei

- der Prozessor (3) dazu konfiguriert ist, basierend auf dem ersten Parameter (P1), dem zweiten Parameter (P2) und dem Parameter zerebraler elektrischer Aktivität (P3) einen ersten Wahrscheinlichkeitsvektor (PV1) zu berechnen, um das Schlaganfallrisiko der Person zu bestimmen, wobei

der erste Wahrscheinlichkeitsvektor (PV1) mindestens zwei Einträge umfasst, die mindestens zwei Wahrscheinlichkeiten mindestens zweier Klassen hinsichtlich des Schlaganfallrisikos angeben.

**10.** System (1) nach Anspruch 9, wobei

- für jeden Parameter des ersten Parameters (P1), des zweiten Parameters (P2) und des Parameters zerebraler elektrischer Aktivität (P3) der Prozessor (3) dazu konfiguriert ist, basierend auf dem Parameter (P1; P2; P3) einen jeweiligen zweiten Wahrscheinlichkeitsvektor (PV2_1; PV2_2; PV2_3) unter Verwendung eines jeweiligen Klassifikators (CL) zu berechnen, um drei zweite Wahrscheinlichkeitsvektoren (PV2_1, PV2_2, PV2_3) zu berechnen, wobei

jeder der drei zweiten Wahrscheinlichkeitsvektoren (PV2_1, PV2_2, PV2_3) mindestens zwei Einträge umfasst, welche die mindestens zwei Wahrscheinlichkeiten der mindestens zwei Klassen hinsichtlich des Schlaganfallrisikos angeben, und
- der Prozessor (3) dazu konfiguriert ist, den ersten Wahrscheinlichkeitsvektor (PV1) durch Kombinieren der drei zweiten Wahrscheinlichkeitsvektoren (PV2_1, PV2_2, PV2_3) unter Verwendung einer gewichteten Summe zu berechnen.

11. System (1) nach Anspruch 10, wobei

- der Prozessor (3) dazu konfiguriert ist, den ersten Wahrscheinlichkeitsvektor (PV1) unter Verwendung eines trainierten Maschinenlernmodells (8) zu berechnen, das jeden der drei zweiten Wahrscheinlichkeitsvektoren (PV2_1, PV2_2, PV2_3) gewichtet und die drei gewichteten zweiten Wahrscheinlichkeitsvektoren summiert, um den ersten Wahrscheinlichkeitsvektor (PV1) zu erzeugen, wobei
- das Maschinenlernmodell unter Verwendung von Trainingsdaten, umfassend eine Vielzahl von Datensätzen, trainiert wird, wobei jeder Datensatz Folgendes umfasst:

- für ein jeweiliges Zeitsegment einen oder mehrere Abtastwerte des ersten Parameters (P1), des zweiten Parameters (P2) und des Parameters zerebraler elektrischer Aktivität (P3), die einem Wahrscheinlichkeitsvektor zugeordnet sind, der mindestens ein bekanntes Schlaganfallrisiko für das jeweilige Zeitsegment angibt.

12. System (1) nach Anspruch 9, wobei

- für jeden Parameter des ersten Parameters (P1), des zweiten Parameters (P2) und des Parameters zerebraler elektrischer Aktivität (P3) der Prozessor (3) dazu konfiguriert ist, basierend auf dem Parameter (P1; P2; P3) zwei oder mehrere jeweilige zweite Wahrscheinlichkeitsvektoren (PV2_$1_1$, PV2_$1_n$; PV2_$2_1$, PV2_$2_n$; PV2_$3_1$, PV2_$3_n$) unter Verwendung zweier oder mehrerer jeweiliger Klassifikatoren (CL_$1$, CL_$n$) zu berechnen, wobei die zwei oder die mehreren jeweiligen Klassifikatoren (CL_$1$, CL_$n$) zwei oder mehreren Klassifikatorarten entsprechen,
- für jede Klassifikatorart der zwei oder der mehreren Klassifikatorarten (CL_$1$, CL_$n$) der Prozessor dazu konfiguriert ist, durch Kombinieren dreier jeweiliger zweiter Wahrscheinlichkeitsvektoren (PV2_$1_1$, PV2_$2_1$, PV2_$3_1$; PV2_$1_n$, PV2_$2_n$, PV2_$3_n$), die durch die Klassifikatorart (CL_$1$; CL_$n$) berechnet werden, unter Verwendung einer gewichteten Summe einen jeweiligen dritten Wahrscheinlichkeitsvektor (PV3_1; PV3_n) zu berechnen, um zwei oder mehrere dritte Wahrscheinlichkeitsvektoren (PV3_1, PV3_n) zu berechnen, und
- der Prozessor (3) dazu konfiguriert ist, den ersten Wahrscheinlichkeitsvektor (PV1) durch Kombinieren der zwei oder der mehreren dritten Wahrscheinlichkeitsvektoren (PV3_1, PV3_n) unter Verwendung einer gewichteten Summe zu berechnen.

13. System (1) nach Anspruch 12, wobei

- der Prozessor (1) dazu konfiguriert ist, den ersten Wahrscheinlichkeitsvektor (PV1) unter Verwendung zweier trainierter Maschinenlernmodelle (81, 82) zu berechnen, wobei für jede Klassifikatorart (CL_$1$; CL_$n$) ein erstes trainiertes Maschinenlernmodell (81) der zwei Modelle (81, 82) jeden der drei jeweiligen zweiten Wahrscheinlichkeitsvektoren (PV2_$1_1$, PV2_$2_1$, PV2_$3_1$; PV2_$1_n$, PV2_$2_n$, PV2_$3_n$) gewichtet und die drei gewichteten zweiten Wahrscheinlichkeitsvektoren summiert, um den jeweiligen dritten Wahrscheinlichkeitsvektor (PV3_1; PV3_n) zu erzeugen,
ein zweites trainiertes Maschinenlernmodell (82) der zwei Modelle (81, 82) jeden der zwei oder der mehreren dritten Wahrscheinlichkeitsvektoren (PV3_1, PV3_n) gewichtet und die zwei oder die mehreren gewichteten dritten Wahrscheinlichkeitsvektoren summiert, um den ersten Wahrscheinlichkeitsvektor (PV1) zu erzeugen, und
- die zwei Maschinenlernmodelle (81, 82) unter Verwendung von Trainingsdaten, umfassend eine Vielzahl von Datensätzen, trainiert werden, wobei jeder Datensatz Folgendes umfasst:

- für ein jeweiliges Zeitsegment einen oder mehrere Abtastwerte des ersten Parameters (P1), des zweiten Parameters (P2) und des Parameters zerebraler elektrischer Aktivität (P3), die einem Wahrscheinlichkeitsvektor zugeordnet sind, der mindestens ein bekanntes Schlaganfallrisiko für das jeweilige Zeitsegment angibt.

14. System (1) nach einem der Ansprüche 9 bis 13, wobei

- jeder Wahrscheinlichkeitsvektor zwei Einträge umfasst, wobei ein erster Eintrag der zwei Einträge die Wahrscheinlichkeit keines Schlaganfallrisikos angibt und ein zweiter Eintrag der zwei Einträge die Wahrscheinlichkeit eines Schlaganfallrisikos angibt;

- jeder Wahrscheinlichkeitsvektor drei Einträge umfasst, wobei ein erster Eintrag der drei Einträge die Wahrscheinlichkeit einer Schlaganfallwahrscheinlichkeit angibt, die kleiner als oder gleich einem ersten Wahrscheinlichkeitsschwellenwert ist, ein zweiter Eintrag der drei Einträge die Wahrscheinlichkeit einer Schlaganfallwahrscheinlichkeit angibt, die größer als der erste Wahrscheinlichkeitsschwellenwert und kleiner als oder gleich einem zweiten Wahrscheinlichkeitsschwellenwert ist, und ein dritter Eintrag der drei Einträge die Wahrscheinlichkeit einer Schlaganfallwahrscheinlichkeit angibt, die größer als der zweite Wahrscheinlichkeitsschwellenwert ist; oder

- jeder Wahrscheinlichkeitsvektor vier Einträge umfasst, wobei ein erster Eintrag der vier Einträge die Wahrscheinlichkeit einer Schlaganfallwahrscheinlichkeit angibt, die kleiner als oder gleich einem ersten Wahrscheinlichkeitsschwellenwert ist, ein zweiter Eintrag der vier Einträge die Wahrscheinlichkeit einer Schlaganfallwahrscheinlichkeit angibt, die größer als der erste Wahrscheinlichkeitsschwellenwert und kleiner als oder gleich einem zweiten Wahrscheinlichkeitsschwellenwert ist, ein dritter Eintrag der vier Einträge die Wahrscheinlichkeit einer Schlaganfallwahrscheinlichkeit angibt, die größer als der zweite Wahrscheinlichkeitsschwellenwert ist, und ein vierter Eintrag der vier Einträge die Wahrscheinlichkeit angibt, dass der erste Parameter, der zweite Parameter und/oder der Parameter zerebraler elektrischer Aktivität, die zum Bestimmen des Wahrscheinlichkeitsvektors verwendet wurden, zum Bestimmen des Wahrscheinlichkeitsvektors nicht geeignet waren.

15. System (1) nach einem der vorhergehenden Ansprüche, wobei

- das System (1) eine Benutzerschnittstelle (5) für Folgendes umfasst:

- Aufklären der Person über das bestimmte Schlaganfallrisiko,
- Ermöglichen der Person, Rechenergebnisse des Prozessors zu korrigieren, und/oder
- Ermöglichen der Person, Ground-Truth-Informationen hinsichtlich eines Schlaganfallrisikos einzugeben; und/oder
- das System (1) eine Warnvorrichtung (6) umfasst, die dazu konfiguriert ist, die Person zu warnen, falls das bestimmte Schlaganfallrisiko größer als ein Schwellenwert für das Risiko ist.

**Revendications**

1. Système (1) pour déterminer un risque d'accident vasculaire cérébral pour une personne, le système (1) comprenant

- une pluralité de capteurs (2), pouvant être portés par la personne, et un processeur (3) ; dans lequel
- la pluralité de capteurs (2) est configurée pour réaliser des mesures non invasives sur la personne pour obtenir un premier paramètre (P1) dépendant de la pression artérielle de la personne, un second paramètre (P2) dépendant de l'oxygénation du sang cérébral de la personne, et un paramètre d'activité électrique cérébrale (P3) de la personne ; et
- le processeur (3) est configuré pour
- obtenir, sur la base de résultats des mesures réalisées par la pluralité de capteurs (2), le premier paramètre (P1), le second paramètre (P2) et le paramètre d'activité électrique cérébrale (P3), et
- déterminer le risque d'accident vasculaire cérébral de la personne sur la base du premier paramètre (P1), du second paramètre (P2) et du paramètre d'activité électrique cérébrale (P3).

2. Système (1) selon la revendication 1, dans lequel

- le premier paramètre (P1) correspond à la sensibilité baroréflexe, BRS, de la personne, et
- la pluralité de capteurs (2) comprend au moins un capteur photopléthysmographique, PPG, (21) configuré pour mesurer une variabilité d'intervalle d'impulsion à impulsion, PPV, et un paramètre d'impulsion indicatif de la variabilité de la pression artérielle, la variabilité facultative de la pression artérielle systolique ; dans lequel
- le processeur (3) est configuré pour calculer, sur la base de la PPV et du paramètre indicatif de la variabilité de la pression artérielle, la BRS.

3. Système (1) selon la revendication 2, dans lequel le paramètre d'impulsion indicatif de la variabilité de la pression artérielle correspond à au moins l'un :

- de la pente de montée d'impulsion, PUS ;

- de l'amplitude d'impulsion, PA ;
- de la largeur d'impulsion, PW ; et
- du temps transitoire de pente d'impulsion, PSTT.

4. Système (1) selon l'une quelconque des revendications précédentes, dans lequel

- le second paramètre (P2) correspond à l'indice d'oxymétrie cérébrale, et
- la pluralité de capteurs (2) comprend au moins deux capteurs de spectroscopie proche infrarouge, NIRS, (22) configurés pour mesurer la saturation cérébrale en oxygène, COS, et la pression artérielle moyenne, MAP ; dans lequel
- le processeur (3) est configuré pour calculer, sur la base de la COS et de la MAP, l'indice d'oxymétrie cérébrale.

5. Système (1) selon l'une quelconque des revendications précédentes, dans lequel

- le paramètre d'activité électrique cérébrale (P3) correspond à un score d'encéphalographie, EEG,
- la pluralité de capteurs (2) comprend au moins cinq capteurs EEG (23),
- le processeur (3) est configuré pour calculer, sur la base de résultats de mesures réalisées par les au moins cinq capteurs EEG (23), le score EEG.

6. Système (1) selon la revendication 5, dans lequel le processeur (3) est configuré pour calculer, sur la base des

- résultats des mesures réalisées par les au moins cinq capteurs EEG (23), au moins l'un :

- d'un rapport entre une puissance mesurée d'ondes delta et une puissance mesurée d'ondes alpha, DAR,
- d'un rapport entre une somme de la puissance mesurée d'ondes delta et une puissance mesurée d'ondes thêta et une somme de la puissance mesurée d'ondes alpha et une puissance mesurée d'ondes bêta, DBATR, et
- d'un indice de symétrie cérébrale dérivé par paires, PDBSI ; et
- le processeur (3) est configuré pour calculer, sur la base d'au moins l'un du DAR, du DBATR et du PDBSI, le score EEG.

7. Système (1) selon l'une quelconque des revendications précédentes, dans lequel

- la pluralité de capteurs (2) comprend au moins un capteur inertiel (24, 24a, 24b), et
- le processeur (3) est configuré pour détecter, sur la base de résultats de mesures réalisées par l'au moins un capteur inertiel (24, 24a, 24b), l'activité de la personne et rejeter le premier paramètre (P1), le second paramètre (P2) et le paramètre d'activité électrique cérébrale (P3) obtenus, en cas de détection d'activité de la personne.

8. Système (1) selon l'une quelconque des revendications précédentes, dans lequel le processeur (3) est configuré pour

- obtenir un score clinique de patient (4) de la personne, et
- déterminer le risque d'accident vasculaire cérébral de la personne sur la base du score clinique de patient de la personne (4) en plus du premier paramètre (P1), du second paramètre (P2) et du paramètre d'activité électrique cérébrale (P3).

9. Système (1) selon l'une quelconque des revendications précédentes, dans lequel

- le processeur (3) est configuré pour calculer, sur la base du premier paramètre (P1), du second paramètre (P2) et du paramètre d'activité électrique cérébrale (P3), un premier vecteur de probabilité (PV1) pour déterminer le risque d'accident vasculaire cérébral de la personne, dans lequel

le premier vecteur de probabilité (PV1) comprend au moins deux entrées indiquant au moins deux probabilités d'au moins deux classes en ce qui concerne le risque d'accident vasculaire cérébral.

10. Système (1) selon la revendication 9, dans lequel

- pour chaque paramètre du premier paramètre (P1), du second paramètre (P2) et du paramètre d'activité électrique cérébrale (P3), le processeur (3) est configuré pour calculer, sur la base du paramètre (P1 ; P2 ; P3), un deuxième vecteur de probabilité respectif (PV2_1 ; PV2_2 ; PV2_3) à l'aide d'un classificateur respectif (CL), pour calculer trois deuxièmes vecteurs de probabilité (PV2_1, PV2_2, PV2_3), dans lequel

chacun des trois deuxièmes vecteurs de probabilité (PV2_1, PV2_2, PV2_3) comprend au moins deux entrées indiquant les au moins deux probabilités des au moins deux classes en ce qui concerne le risque d'accident vasculaire cérébral, et

- le processeur (3) est configuré pour calculer le premier vecteur de probabilité (PV1) en combinant les trois deuxièmes vecteurs de probabilité (PV2_1, PV2_2, PV2_3) à l'aide d'une somme pondérée.

**11.** Système (1) selon la revendication 10, dans lequel

- le processeur (3) est configuré pour calculer le premier vecteur de probabilité (PV1) à l'aide d'un modèle d'apprentissage automatique entraîné (8) qui pondère chacun des trois deuxièmes vecteurs de probabilité (PV2_1, PV2_2, PV2_3) et additionne les trois deuxièmes vecteurs de probabilité pondérés pour générer le premier vecteur de probabilité (PV1), dans lequel

- le modèle d'apprentissage automatique est entraîné à l'aide de données d'entraînement comprenant une pluralité d'ensembles de données, dans lequel chaque ensemble de données comprend

- pour un segment de temps respectif, un ou plusieurs échantillons du premier paramètre (P1), du second paramètre (P2) et du paramètre d'activité électrique cérébrale (P3) associés à un vecteur de probabilité indiquant au moins un risque connu d'accident vasculaire cérébral pour le segment de temps respectif.

**12.** Système (1) selon la revendication 9, dans lequel

- pour chaque paramètre du premier paramètre (P1), du second paramètre (P2) et du paramètre d'activité électrique cérébrale (P3), le processeur (3) est configuré pour calculer, sur la base du paramètre (P1 ; P2 ; P3), deux ou plusieurs deuxièmes vecteurs de probabilité respectifs (PV2_1$_1$, PV2_1$_n$; PV2_2$_1$, PV2_2$_n$; PV2_3$_1$, PV2_3$_n$) à l'aide de deux ou plusieurs classificateurs respectifs (CL$_1$, CL$_n$), dans lequel les deux ou plusieurs respectifs (CL$_1$, CL$_n$) correspondant à deux ou plusieurs types de classificateurs,

- pour chaque type de classificateur des deux ou plusieurs types de classificateurs (CL$_1$, CL$_n$), le processeur est configuré pour calculer un troisième vecteur de probabilité respectif (PV3_1 ; PV3_n) en combinant trois deuxièmes vecteurs de probabilité respectifs (PV2_1$_1$, PV2_2$_1$, PV2_3$_1$; PV2_1$_n$, PV2_2$_n$, PV2_3$_n$), calculés par le type de classificateur (CL$_1$; CL$_n$), à l'aide d'une somme pondérée pour calculer deux ou plusieurs troisièmes vecteurs de probabilité (PV3_1, PV3_n), et

- le processeur (3) est configuré pour calculer le premier vecteur de probabilité (PV1) en combinant les deux ou plusieurs troisièmes vecteurs de probabilité (PV3_1, PV3_n) à l'aide d'une somme pondérée.

**13.** Système (1) selon la revendication 12, dans lequel

- le processeur (1) est configuré pour calculer le premier vecteur de probabilité (PV1) à l'aide de deux modèles d'apprentissage automatique entraînés (81, 82), dans lequel

pour chaque type de classificateur (CL$_1$ ; CL$_n$), un premier modèle d'apprentissage automatique entraîné (81) des deux modèles (81, 82) pondère chacun des trois deuxièmes vecteurs de probabilité respectifs (PV2_1$_1$, PV2_2$_1$, PV2_3$_1$ ; PV2_1$_n$, PV2_2$_n$, PV2_3$_n$) et additionne les trois deuxièmes vecteurs de probabilité pondérés pour générer le troisième vecteur de probabilité respectif (PV3_1 ; PV3_n),

un second modèle d'apprentissage automatique entraîné (82) des deux modèles (81, 82) pondère chacun des deux ou plusieurs troisièmes vecteurs de probabilité (PV3_1, PV3_n) et additionne les deux ou plusieurs troisièmes vecteurs de probabilité pondérés pour générer le premier vecteur de probabilité (PV1), et

- les deux modèles d'apprentissage automatique (81, 82) sont entraînés à l'aide de données d'entraînement comprenant une pluralité d'ensembles de données, dans lequel chaque ensemble de données comprend

- pour un segment de temps respectif, un ou plusieurs échantillons du premier paramètre (P1), du second paramètre (P2) et du paramètre d'activité électrique cérébrale (P3) associés à un vecteur de probabilité indiquant au moins un risque connu d'accident vasculaire cérébral pour le segment de temps respectif.

**14.** Système (1) selon l'une quelconque des revendications 9 à 13, dans lequel

- chaque vecteur de probabilité comprend deux entrées, dans lequel une première entrée des deux entrées indique la probabilité d'absence de risque d'accident vasculaire cérébral et une seconde entrée des deux entrées indique la probabilité d'un risque d'accident vasculaire cérébral ;
- chaque vecteur de probabilité comprend trois entrées, dans lequel une première entrée des trois entrées indique la probabilité d'une probabilité d'accident vasculaire cérébral inférieure ou égale à un premier seuil de probabilité, une deuxième entrée des trois entrées indique la probabilité d'une probabilité d'accident vasculaire cérébral supérieure au premier seuil de probabilité et inférieure ou égale à un deuxième seuil de probabilité et une troisième entrée des trois entrées indique la probabilité d'une probabilité d'accident vasculaire cérébral supérieure au deuxième seuil de probabilité ; ou
- chaque vecteur de probabilité comprend quatre entrées, dans lequel une première entrée des quatre entrées indique la probabilité d'une probabilité d'accident vasculaire cérébral inférieure ou égale à un premier seuil de probabilité, une deuxième entrée des quatre entrées indique la probabilité d'une probabilité d'accident vasculaire cérébral supérieure au premier seuil de probabilité et inférieure ou égale à un deuxième seuil de probabilité, une troisième entrée des quatre entrées indique la probabilité d'une probabilité d'accident vasculaire cérébral supérieure au deuxième seuil de probabilité, et une quatrième entrée des quatre entrées indique la probabilité que le premier paramètre, le second paramètre et/ou le paramètre d'activité électrique cérébrale, utilisés pour déterminer le vecteur de probabilité, n'étaient pas adaptés pour déterminer le vecteur de probabilité.

15. Système (1) selon l'une quelconque des revendications précédentes, dans lequel

- le système (1) comprend une interface utilisateur (5) pour
- informer la personne du risque déterminé d'accident vasculaire cérébral,
- permettre à la personne de corriger les résultats de calcul du processeur, et/ou
- permettre à la personne de saisir des informations de vérité terrain concernant un risque d'accident vasculaire cérébral ; et/ou
- le système (1) comprend un dispositif d'alerte (6) configuré pour alerter la personne dans le cas où le risque déterminé d'accident vasculaire cérébral est supérieur à un seuil pour le risque.

**Figure 1**

Figure 2

Figure 3

Figure 4

**Figure 5**

**Figure 6**

**Figure 7**

EP 4 319 618 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2021106238 A